# EUROPEAN PATENT APPLICATION

(11) **EP 1 146 453 A2**
(43) Date of publication of application: **17.10.2001**
(21) Application number: 01107262.6
(22) Date of filing: 23.03.2001
(51) Int. Cl.: G06F 17/60

(54) **Data storage, management, and delivery method**

(30) Priority: 31.03.2000 JP 2000100032; 31.03.2000 JP 2000100033
(71) Applicant: MATSUSHITA ELECTRIC INDUSTRIAL CO., LTD., Kadoma-shi, Osaka 571-8501 (JP)
(72) Inventor: Imanaka, Ryoichi, Hirakata-shi, Osaka 573-1112 (JP); Ito, Nobuaki, Neyagawa-shi, Osaka 572-0087 (JP); Suyama, Satoshi, Nishinomiya-shi, Hyogo 663-8005 (JP); Furuse, Tsukasa, Ishikari-shi, Hokkaido 061-3211 (JP); Hara, Makoto, Paaku-puraza D-202, Sapporo-shi, Hokkaido 004-0054 (JP)
(74) Representative: Dr. Elisabeth Jung Dr. Jürgen Schirdewahn Dipl.-Ing. Claus Gernhardt

(57) **Abstract**

An object or information management method is provided that enables a customer (1) to easily and quickly retrieve information contained in a stored object. The object or information management method comprises: a storing step for storing an object of two-dimensional or three-dimensional shape received from the customer; a first storing step for storing in a storage device a first identifier for identifying the customer and a second identifier for identifying the object by associating the first and second identifiers with the object; an accepting step for accepting a request from the customer requesting retrieval of the object, the request containing therein the first identifier and the second identifier; a step for comparing the first identifier contained in the request with the first identifier stored in the storage device, and for rejecting the request if the two first identifiers are not substantially the same; a searching step for searching for the object based at least on the second identifier; a converting step for converting information contained in the object into first information which is analog data or digital data; and a sending step for sending the first information to the customer.

## Description

### BACKGROUND OF THE INVENTION

The present invention relates to the field of object or information storage business.

Hospitals, medical clinics, and other medical institutions, in their daily medical activities, produce objects having two-dimensional shapes such as chest X-rays of tuberculosis patients and photographs of affected sites of patients with burns, objects having three-dimensional shapes such as dental dies taken from patients with dental caries, or information (intangibles) such as X-ray tomographic image (CT scanned image) data of patients with brain tumors.

Hospitals and like institutions use these X-ray photographs, etc. in the diagnosis of diseases, but once the diagnosis is done, the photographs, etc. will not be used for some time.

However, there are cases where, when examining a patient after watching the progress the patient has made, the doctor may want to take a new X-ray photograph and compare it with a previously taken X-ray photograph. Such X-ray photographs can also be used as physical evidence, for example, in the case of a damage suit for malpractice, etc.

For these and other purposes, hospitals and like institutions must store the X-ray photographs, etc.

However, at hospitals where the staff are kept busy with medical treatment and other work, it is extremely difficult to perfectly manage information and objects such as X-ray photographs being produced and piling up in their daily medical activities.

The present invention relates to a method of managing objects such as X-ray photographs and information such as X-ray tomographic image data that hospitals generate in their medical activities.

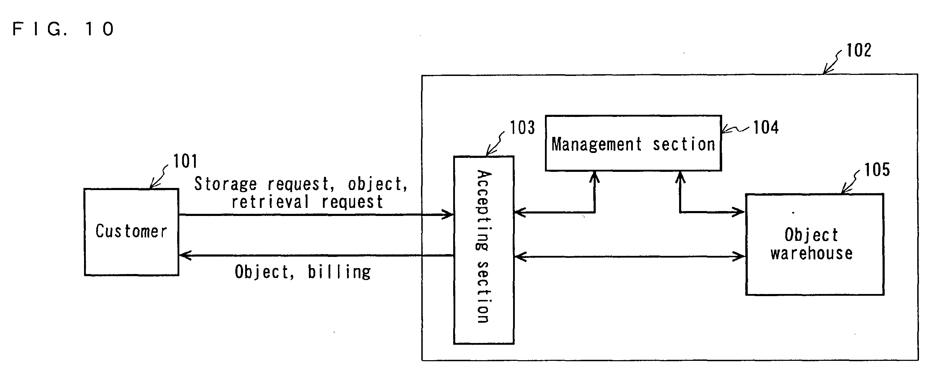

Figure 10 illustrates an object management method according to the prior art.

Customer 101 takes or sends an object to a storage system (for example, a storage company) 102 to which he or she desires to entrust the management of the object, and requests the storage of the object.

An accepting section 103 in the storage system 102 identifies the customer's name and the object to be stored, and accepts the request.

The accepting section 103 sends information including the customer's name, the object to be stored, and the date of acceptance, to a management section 104 which then records and stores these pieces of information on a recording device (not shown).

On the other hand, the accepting section 103 stores the object in an object warehouse 105.

When the customer 101 makes a request to the accepting section 103 to retrieve the stored object, the accepting section 103 identifies the customer's name and the stored object, and accepts the request. Next, the accepting section 103 directs the object warehouse 105 to retrieve the object. The object warehouse 105 retrieves the requested object and passes it to the accepting section 103. The accepting section 103 delivers the object to the customer 101.

The management section 104 calculates the storage fee, and charges the customer 101 for the storage through the accepting section 103.

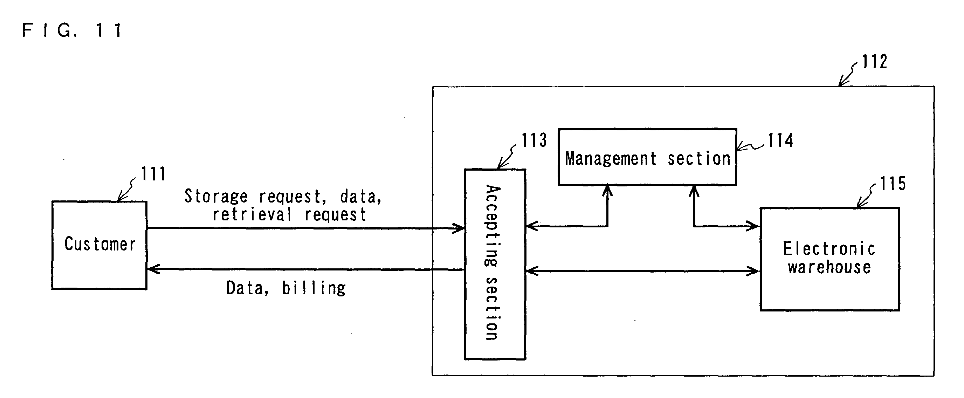

Figure 11 illustrates an information (intangibles such as analog data or digital data) management method according to the prior art.

Customer 111 sends a request for storage and information the storage of which he or she desires to entrust, to a storage system (for example, a storage company) 112 via the Internet or the like.

An accepting section 113 in the storage system 112 identifies the customer's name and the information to be stored, and accepts the request.

The accepting section 113 sends information including the customer's name, the information to be stored, and the date of acceptance, to a management section 114 which then records and stores these pieces of information on a recording device (not shown).

On the other hand, the accepting section 113 stores the information received from the customer in an electronic warehouse 115.

When the customer 111 makes a request to the accepting section 113 to retrieve the stored information, the accepting section 113 identifies the customer's name and the stored information, and accepts the request. Next, the accepting section 113 directs the electronic warehouse 115 to retrieve the requested information. The electronic warehouse 115 retrieves the information and passes it to the accepting section 113. The accepting section 113 delivers the information to the customer 111.

The management section 114 calculates the storage fee, and charges the customer 111 for the storage through the accepting section 113.

Consider the case where a hospital as a customer entrusts the storage of objects, in this case, X-ray photographs of patients. Suppose, for example, that a doctor at the hospital, who is examining a patient, wants to see a previously taken X-ray photograph.

In the prior art, however, if the doctor telephones the storage company (for example, a warehouse company) and requests them to retrieve the X-ray photograph, the doctor would end up being unable to examine the requested X-ray photograph in the presence of the patient, because it takes some time to retrieve the photograph from the warehouse and to send it to the hospital.

On the other hand, when a hospital takes an X-ray photograph of a patient and entrusts the storage of the photograph to a storage company, the general tendency is that it often becomes necessary to review the X-ray photograph shortly after (for example, one week after) the photograph was taken, while it seldom becomes necessary to review the X-ray photograph long after (for example, three years after) the photograph was taken.

In the prior art method, however, when the storage of an X-ray photograph is entrusted to a storage company, it has been practiced to immediately and indiscriminately transfer the X-ray photograph for storage in a warehouse far from the hospital.

When the storage of an object is entrusted by a customer, the storage company assigns an identifier (typically, an identification number) to the customer as well as to the object to be stored, attaches a label carrying the identifier to the stored object, and enters the identifier into the management section, a computer, for storing therein. Accordingly, when the customer requests the retrieval of the stored object, usually the requested object precisely is delivered.

By contrast, in the present invention to be described in detail later, when the customer requests the retrieval of a stored object, first information generated by extracting information contained in the object is transmitted to the customer.

However, when the customer requested the delivery of the chest X-ray photograph of a patent P, for example, if the chest X-ray photograph of a patent Q was delivered by mistake, this could endanger the life of the patient because generally it is difficult to distinguish between the two photographs.

Furthermore, in the prior art management method, the management of objects and the management of information have been handled totally independently of each other.

There are cases, however, where the hospital entrusts the storage of an object, for example, an X-ray photograph of a patient, and the storage of information, i.e., the clinical chart of that patient (information produced by entering the clinical chart into a computer), to the storage company.

However, in the prior art, since the storage of objects and the storage of information have been managed independently of each other, the customer has not been able to view a storage list consolidating, for example, an object storage list and an information storage list.

Consider the case where a hospital as the customer entrusts the storage of objects, such as X-ray photographs of patients. Suppose, for example, that a doctor at the hospital took an X-ray tomographic image of a patient and analyzed the data, but that the doctor wants an experienced doctor outside the hospital to analyze the same X-ray tomographic image data to make sure.

In the prior art, the doctor would find an experienced doctor through his personal connections and send the image data to the experienced doctor for analysis. The experienced doctor would analyze the received image data, prepare a medical certificate, and return the image data together with the medical certificate.

The doctor at the hospital would have to pay a fee to the experienced doctor.

In the prior art, the doctor has had to do the time consuming job just described above. It has also been difficult to find an experienced doctor.

Likewise, there are cases where a patent would like his or her X-ray tomographic image data to be analyzed by a doctor other than the doctor at the hospital he or she is visiting regularly. In the prior art, it has been extremely difficult to fulfill such wishes.

Further, when a patient is transferred to another hospital, the medical treatment records of the patient must also be transferred from the previous hospital to the new hospital.

However, if the new hospital is to be given the opportunity to view the previously taken X-ray photographs, etc . of the patient, the parties concerned have had to meet in person, bringing the actual X-ray photographs, etc.

If the new hospital wishes to take duplicates of the X-ray photographs, etc., it has been difficult to produce duplicates.

Furthermore, when a patient is transferred to another hospital, the new hospital has been able to request that the new hospital be given the opportunity to view data such as the X-ray photographs, etc. produced at the previous hospital, but if the patient wishes his or her X-ray photographs to be inspected by the new hospital, he or she alone has not been able to give the new hospital the opportunity to inspect them.

Further, when a patient wishes his or her X-ray tomographic image data to be analyzed by a doctor outside the hospital where the patient is hospitalized, the patient has had no other way but to ask the doctor through his or her doctor at the hospital.

### SUMMARY OF THE INVENTION

According to the invention described in claim 1, there is provided an object or information management method comprising:
a storing step for storing an object of two-dimensional or three-dimensional shape received from a customer;
a first storing step for storing in a storage device a first identifier for identifying the customer and a second identifier for identifying the object by associating the first and second identifiers with the object;
an accepting step for accepting a request from the customer requesting retrieval of the object, the request containing therein the first identifier and the second identifier;
a step for comparing the first identifier contained in the request with the first identifier stored in the storage device, and for rejecting the request if the two first identifiers are not substantially the same;
a searching step for searching for the object based at least on the second identifier;
a converting step for converting information contained in the object into first information which is analog data or digital data; and
a sending step for sending the first information to the customer.

In the prior art, if the customer entrusted the storage of an object and requested at a later date the retrieval of the object, the customer was unable to view the object until it was delivered to the customer by mail or other means.

In the present invention, when the customer who entrusted the storage of an object makes a request for retrieval of the stored object, the information contained in the object is converted into the first information, and the first information is sent to the customer via the Internet or the like, rather than sending the object itself to the customer.

Thus, the invention offers the effect of achieving a management method that permits the customer to easily and quickly retrieve the information contained in the object entrusted for storage.

In the prior art management method, the cost of retrieval has been high because the object itself has had to be delivered from the storage warehouse to the customer and, after finishing the inspection of the object, the customer has had to return the object to the warehouse.

The present invention offers the effect of achieving a management method that can send the retrieved information from the warehouse to the customer at extremely low cost, particularly, by using a communication line such as the Internet, and that eliminates the need for the customer to return the information (no cost for return is incurred).

The converting step may be performed after receiving the object from the customer and before accepting the retrieval request from the customer. Alternatively, the converting step may be performed after accepting the retrieval request from the customer.

Preferably, the converting step is performed after accepting the retrieval request from the customer. This is because the customer does not always request the retrieval of the first information of the object after the object has been entrusted for storage; therefore, by converting the object into the first information only when the retrieval request is received, the amount of conversion work as a whole can be reduced, and the storage labor and expenses can thus be saved.

The wording "the two first identifiers are substantially the same" means, for example, that when a doctor F at a hospital A employing many doctors sends a request for the retrieval of an X-ray photograph by attaching the customer ID of the hospital A and the personal ID of the doctor, the retrieval request received from the doctor F is granted as long as the customer ID attached to the request matches the customer ID of the hospital A carried in a storage list (the personal ID of the doctor F is not checked).

The term "information contained in the object" means, for example, image information of an X-ray photograph.

After the first information is transmitted to the customer, the first information may be left stored in the storage device or may be erased from the storage device. Generally, it is preferable to leave the first information stored in the storage device.

The present invention is applicable for storage of any kind of object, but is particularly suited as a storage method for storing data generated in the course of medical treatment and examination or preventive activities.

In the medical field, a great deal of data are generated in daily medical treatment and examination or preventive activities, and at hospital and like institutions where professionals of medical treatment work in groups, there is an increasing demand to entrust the storage of such data to an outside source. There are, however, cases where after data (for example, an object such as an X-ray photograph or information such as CT scanned image data) has been stored in an outside warehouse, a doctor who is seeing a patient may want to compare it with a previously taken X-ray photograph. In such cases, it is desired that the image data of the X-ray photograph stored in the warehouse be immediately retrieved for display on the terminal display at the hospital (there is no need to retrieve the X-ray photograph itself).

While the prior art storage method has not been able to meet such needs, the present invention offers the effect of achieving an object or information management method that can meet such needs.

According to the invention described in claim 2, there is provided an object or information management method comprising: a storing step for storing an object of two-dimensional or three-dimensional shape received from a customer, or for storing second information received from a customer in a storage device, the second information being analog data or digital data;
a first storing step for storing in a storage device a first identifier for identifying the customer and a second identifier for identifying the object or the second information;
an accepting step for accepting a request from the customer requesting retrieval of the object or the second information, the request containing therein the first identifier and the second identifier;
a step for comparing the first identifier contained in the request with the first identifier stored in the storage device, and for rejecting the request if the two first identifiers are not substantially the same;
a searching step for searching for the object or the second information based at least on the second identifier;
a converting step for converting information contained in the object into first information which is analog data or digital data, the converting step being performed when the customer is requesting the retrieval of the object; and
a sending step for sending the first information or the second information to the customer.

In the prior art, the storage of objects and the storage of information were managed independently of each other. Therefore, the hospital entrusted, for example, a leg photograph of a patient S with burns for storage in the object warehouse, and the electronic clinical chart of the same patient S for storage in the electronic warehouse. Since the data generated in the course of the treatment of the patient S were stored separately in the two warehouses, there was no such list that consolidated all data concerning the patient S. This made it difficult for the hospital to compare and study the data, and sometimes led to the possibility of creating duplicate data.

The present invention offers the effect of being able to achieve a method for storing objects and information in a consolidating manner. As a result, the hospital can obtain a list of all the data (including objects and information). concerning the patient S, and can thus easily compare and study the data created in the past.

In the prior art, if the customer entrusted the storage of an object and requested at a later date the retrieval of the object, the customer was unable to view the object until it was delivered to the customer by mail or other means.

In the present invention, when the customer who entrusted the storage of an object makes a request for retrieval of the stored object, the information contained in the object is converted into the first information, and the first information is sent to the customer via the Internet or the like, rather than sending the object itself to the customer.

Thus, the invention offers the effect of achieving a management method that permits the customer to easily and quickly retrieve the information contained in the object entrusted for storage.

Furthermore, since the first information generated at this time is stored in the information warehouse (electronic warehouse), when the customer requests the retrieval of the same object for a second time, the first information stored in the electronic warehouse can be immediately transmitted to the customer without again converting the object into the first information. This saves the labor and time that would otherwise have to be expended for the conversion.

The invention thus offers the effect of achieving an efficient object or information management method by linking the object warehouse and the electronic warehouse in coordinating fashion.

In the prior art management method, the cost of retrieval has been high because the object itself has had to be delivered from the storage warehouse to the customer and, after finishing the examination of the object, the customer has had to return the object to the warehouse.

The present invention offers the effect of achieving a management method that can send the retrieved information from the warehouse to the customer at extremely low cost, particularly, by using a communication line such as the Internet, and that eliminates the need for the customer to return the information (no cost for return is incurred).

The invention described in claim 3 concerns the object or information management method as described in claim 1 or 2, wherein the method further includes the step of presenting the customer with an image display of a storage list showing both the stored object and the stored second information.

The present invention thus offers the effect of being able to achieve a method for storing objects and information in a consolidating manner. As a result, the hospital can obtain a list of all the data (including objects and information) concerning the patient S, and can thus easily compare and study the data created in the past.

The invention described in claim 4 concerns the object or information management method as described in claim 1 or 2, wherein the accepting in the accepting step and the sending in the sending step are each performed by transmitting a digital data sequence over a communication line.

The invention thus offers the effect of achieving an object or information management method that enables high speed communications between the customer and the remotely located storage system (including a warehouse) using a communication line, thereby allowing the customer to quickly retrieve the stored information, etc.

The invention described in claim 5 concerns the object or information management method as described in claim 4, wherein the digital data sequence is encrypted.

The invention thus offers the effect of achieving an object or information management method that can ensure secrecy of customer information by encrypting the data for transmission.

The invention described in claim 6 concerns the information management method as described in claim 1 or 2, wherein
the first information includes therein at least one of visual indications of customer name, customer identifier, attribute information of the object, identifier of the object, or an optical identifier having information consisting of at least one of the first and second identifiers, or
the second information includes therein at least one of visual indications of customer name, customer identifier, attribute information of the second information, identifier of the second information, or an optical identifier having information consisting of at least one of the first and second identifiers.

By displaying the stored information, etc. on a terminal screen, the customer can visually confirm that the received information is precisely the requested information, etc.

In the object or information method for storing, in particular, data that hospitals, etc. generate in the course of their medical treatment and examination or preventive activities, it is extremely important to verify the correctness of information because wrongly identifying information could endanger the life of the patient.

The invention offers the effect of achieving an object or information management method that can easily verify the correctness of the received information by visual means.

The optical identifier is, for example, a bar code.

The invention described in claim 7 concerns the information management method as described in claim 1 or 2, wherein the method further includes:
a step for counting the number of times that the customer retrieves the object, the first information, or the second information; and
a step for billing the customer for charges including a charge corresponding to the number of retrievals.

The invention offers the effect of being able to achieve the object or information management method including the billing method.

The invention described in claim 8 concerns the information management method as described in claim 1 or 2, wherein
when the information contained in the object is converted into the first information, the method further includes:
a second storing step for storing the first information in a storage device; and
a step for storing third information in a storage device by associating the third information with the object, the third information providing an indication of whether the first information into which the information contained in the object has been converted is stored or not, and wherein:
   the searching step searches for the first information when the third information associated with the object to be searched for indicates that the first information is stored, and
   when the first information is stored, the sending step reads out the stored first information and sends the readout first information.

The invention pertains to an object or information management method wherein when the retrieval of an object is requested for the first time, the object is converted into first information and the first information is stored in the electronic warehouse for retrieval in the future.

This offers the effect of being able to achieve an efficient object or information management method wherein when the first information is already stored, the first information, rather than the object itself, is retrieved from the warehouse (except when the retrieval of the object itself is requested), thereby eliminating the need to repeat the same conversion work (the generation of the first information).

The invention described in claim 9 concerns the information management method as described in claim 1 or 2, wherein
when the information contained in the object is converted into the first information, the method further includes a second storing step for storing the first information in a storage device, and wherein:
the searching step first searches for the first information and, if the first information is not found, then searches for the object, and
when the first information is stored, the sending step reads out the stored first information and sends the readout first information.

The invention pertains to an object or information management method in which once the retrieval of an object is requested, the object is converted into first information and the first information is stored in the electronic warehouse.

This offers the effect of being able to achieve an efficient object or information management method wherein when the first information is already stored, the first information, rather than the object itself, is retrieved from the warehouse (except when the retrieval of the object itself is requested), thereby eliminating the need to repeat the same conversion work (the generation of the first information).

The invention described in claim 10 concerns the information management method as described in claim 1 or 2, wherein
when the object is already received from the customer, the accepting step allows the customer to select and specify whether the sending step should send the object or the first information,
when the customer specifies that the object be sent, the sending step sends the object to the customer,
when the customer specifies that the first information be sent, the sending step sends the first information to the customer, and
when the customer requests that the sending step send the object only, the converting step is not performed.

In the object or information management method of the invention, the customer who entrusted the storage of an object to a storage company or the like can select whether to retrieve the object itself or the information contained in the object. For example, when it is necessary to submit an X-ray photograph as physical evidence in a malpractice suit or the like, the customer can request the retrieval of the object itself, while when, for example, it is desired to review a previously taken X-ray photograph on the spot, the customer can request the retrieval of the first information for immediate delivery.

The invention thus offers the effect of achieving an object or information management method that allows the customer to select the object or information for retrieval according to the needs of the customer.

When the customer requests the retrieval of the object itself, the conversion work (the generation of the first information) is not performed. This offers the effect of achieving an efficient object or information management method by avoiding time-wasting work.

The invention described in claim 11 concerns the information management method as described in claim 1 or 2, wherein the method further includes:
a step for accepting a request from the customer for return of the object;
a step for returning the object to the customer;
a step for searching for the first information corresponding to the object; and
a step for erasing the first information if the first information is stored.

The ownership of the stored object belongs to the customer, and the storage company is bound to secrecy. In particular, it is important to protect the secrecy of objects, etc. entrusted by a hospital, etc. for storage. The first information is generated under agreement with the customer, but if the first information is kept stored after the object is returned, the secrecy of the customer may not be able to be protected.

The invention thus offers the effect of achieving an object or information management method that can ensure the secrecy of the customer.

According to the invention described in claim 12, there is provided an information management method comprising:
a first storing step for storing second information received from a customer, the second information being analog data or digital data, a first identifier for identifying the customer, and a second identifier for identifying the second information, at an address having first location information in a first place;
a transmitting step for transmitting information including the first identifier, the second identifier, and the first location information, to a management section;
a first responding step for outputting the second information from a storage device located in the first place, if a request containing therein the first identifier and the second identifier and requesting retrieval of the second information is made by the customer;
a step for the management section to issue an instruction to transfer the second information from the first place to a second place when the period of storage in the first place has exceeded a predetermined period, or when the amount of information stored in the storage device in the first place has exceeded a predetermined amount, or when the customer requests the transfer;
a second storing step for storing the second information at an address having second location information in the second place;
a step for the management section to store therein the second location information by associating the second location information with the first identifier and the second identifier; and
a sending step for searching for the second information in accordance with an instruction from the management section, and sending the second information to the customer via a communication line, if a request containing therein the first identifier and the second identifier and requesting retrieval of the second information is received from the customer.

The present invention is applicable for the management of any information, but is particularly effective for the management of information generated in the medical field.

In the case of objects or information generated in the course of medical treatment and examination or preventive activities, the probability of the generated data being retrieved is high shortly after they are generated, but the probability tends to decrease rapidly as the time elapses.

In view of this, in the present invention, data (information) is stored in the first place near the customer such as a hospital for a predetermined period after the generation of the data, and when the predetermined time has elapsed, the data is transferred for storing in the second place which is remotely located from the hospital but has a larger space.

That is, during the period that the probability of retrieval is high, the data is stored in the nearby first place, and when the probability of retrieval has decreased, the data is transferred to the second place having a larger space.

The invention thus offers the effect of achieving an object or information management method that provides enhanced convenience by changing the storage place of information depending on the probability of retrieval.

The starting time of the predetermine period is not specifically limited. For example, the predetermined period may start from the time that an object is received from the customer or the received object is stored in the first place, or from the last time that the object stored in the first place was delivered to the customer at the customer's request or the object delivered to the customer was returned from the customer.

Further, the length of the predetermined period is not specifically limited.

The management section in the first place and the management section in the second place may be the same management section (in this case, the management section is usually installed in the second place), or the management section in the first place and the management section in the second place may be separate entities linked by a communication line.

Whether the computer for recording the second information is a computer installed at the customer or a computer belonging to the management system does not constitute a requirement for the present invention. The present invention concerns a method of information management; therefore, the second information, if it is recorded in the computer at the customer, is the "second information" "received from the customer" according to the definition of the present invention, as long as the second information is managed by the management system. This also applies to other claims of the invention.

According to the invention described in claim 13, there is provided an object or information management method comprising:
a first depositing step for depositing an object received from a customer, or a medium received from the customer and having recorded thereon second information which is analog data or digital data, at an address having first location information in a first place;
a first storing step for transmitting information including a first identifier for identifying the customer, a second identifier for identifying the object or the medium, and the first location information, to a management section, and for storing the information in the management section;
an instructing step for the management section to issue an instruction to transfer the object or the medium from the first place to a second place when the period of deposit in the first place has exceeded a predetermined period, or when the object or the medium stored in the storage device in the first place has exceeded a predetermined quantity, or when the customer requests the transfer;
a second depositing step for transferring the object or the medium from the first place to the second place, and for depositing the object or the medium at an address having second location information in the second place;
a second storing step for the management section to store therein the second location information by associating the second location information with the first identifier and the second identifier; and
a sending step for searching for the object or the medium in accordance with an instruction from the management section, and sending first information, which is analog data or digital data obtained by converting information contained in the object, or the second information reproduced from the medium, to the customer via a communication line, if a request containing therein the first identifier and the second identifier and requesting retrieval of the object or the second information is received from the customer.

The present invention is applicable for the management of any information, but is particularly effective for the management of information generated in the medical field.

In the case of objects or information generated in the course of medical treatment and examination or preventive activities, the probability of the generated data being retrieved is high shortly after they are generated, but the probability tends to decrease rapidly as the time elapses.

In view of this, in the present invention, data (an object or information) is stored in the first place near the customer such as a hospital for a predetermined period after the generation of the data, and when the predetermined time has elapsed, the data is transferred for storing in the second place which is remotely located from the hospital but has a larger space.

That is, during the period that the probability of retrieval is high, the data is stored in the nearby first place, and when the probability of retrieval has decreased, the data is transferred to the second place having a larger space.

The invention thus offers the effect of achieving an object or information management method that provides enhanced convenience by changing the storage place of the object or information depending on the probability of retrieval.

The wording "medium received from the customer and having recorded thereon second information which is analog data or digital data" refers to the case where the first information is recorded on a medium at the customer side, as well as the case where the second information itself is received from the customer and the received second information is recorded on a medium.

The invention described in claim 14 concerns the information management method as described in claim 12 or 13, wherein the management section includes a first computer comprising a communication device installed in the first place, and a second computer comprising a communication device installed in the second place.

The invention offers the effect of achieving an information management method that provides enhanced convenience by changing the storage place of information depending on the probability of retrieval.

The invention described in claim 15 concerns the information management method as described in claim 14, wherein the second computer has a first data space not accessible by the customer and a second data space accessible by the customer, and wherein the method further includes the step of allowing the customer to access data stored in the second data space via the Internet after the first identifier has been identified.

The invention thus offers the effect of achieving an object or information management method that can ensure the secrecy of the customer.

According to the invention described in claim 16, there is provided an object or information management method comprising:
a storing step for storing an object of two-dimensional or three-dimensional shape received from a customer;
a first storing step for storing in a storage device a first identifier for identifying the customer and a second identifier for identifying the object;
an accepting step for accepting a request from the customer requesting analysis of the object, the request containing therein the first identifier and the second identifier;
a step for comparing the first identifier contained in the request with the first identifier stored in the storage device, and for rejecting the request if the two first identifiers are not substantially the same;
a searching step for searching for the object based at least on the second identifier;
a converting step for converting information contained in the object into first information which is analog data or digital data;
a first sending step for sending the first information to an analyzing person;
a receiving step for receiving an analysis result transmitted from the analyzing person; and
a second sending step for sending the analysis result to the customer.

The invention offers the effect of achieving an object or information management method wherein, at the request of the customer, the storage company or the like that stores objects entrusts the analysis of a stored object (for example, an X-ray photograph) to a third party.

The invention thus provides analysis service of, among others, data (objects or information) generated in the course of medical treatment and examination or preventive activities. In recent years, with advances in medical technologies, medical practitioners have become more and more specialized. In this context, if a general doctor can easily obtain a medical certificate from an expert doctor having a high analysis skill (for example, the interpreting of X-ray photographs or tomographic image data), it not only serves in the interest of the customer, but also greatly benefits the patient.

The customer can receive analysis service from an experienced doctor just by specifying the data to be analyzed (for example, an X-ray photograph) and submitting a request to the storage company to request the analysis by a third party (the customer may specify a particular doctor for the analysis).

Bit mapped image data with a deposit ID attached to it is sent to the doctor who performs the analysis, but since any data identifying the individual patient, such as the patient ID or patient's name, is not attached to the image data, the secrecy of the patient can be protected.

The invention thus offers the effect of achieving an object or information management method that can ensure the secrecy of the individual patient.

Since the storage company already has the requested data in the storage and also has a digitizer for X-ray photographs, etc., the storage company can send the data to the experienced doctor in a short time, and the customer can obtain the result of the analysis (medical certificate) in a short time.

The invention offers the effect of achieving an object or information management method that provides the service in which the storage company pays the analysis fee to the doctor on behalf of the customer, together with the fees for analyses the doctor did for other customers.

The customer, on the other hand, pays the analysis fee to the storage company together with the monthly storage charge. This saves the customer the extra time and labor involved in the payment.

"Analysis" means making a diagnosis by examining the data based on expertise. This includes, among others, the interpreting of X-ray photographs, X-ray tomographic image data, and other medical data.

According to the invention described in claim 17, there is provided an object or information management method comprising:
a storing step for storing an object of two-dimensional or three-dimensional shape received from a customer, or for storing second information received from a customer in a storage device, the second information being analog data or digital data;
a first storing step for storing in a storage device a first identifier for identifying the customer and a second identifier for identifying the object or the second information;
an accepting step for accepting a request from the customer requesting analysis of the object or the second information, the request containing therein the first identifier and the second identifier;
a step for comparing the first identifier contained in the request with the first identifier stored in the storage device, and for rejecting the request if the two first identifiers are not substantially the same;
a searching step for searching for the object or the second information based at least on the second identifier;
a converting step for converting information contained in the object into first information which is analog data or digital data, the converting step being performed when the customer is requesting the analysis of the object;
a first sending step for sending the first information or the second information to an analyzing person;
a receiving step for receiving an analysis result transmitted from the analyzing person; and
a second sending step for sending the analysis result to the customer.

The invention offers the effect of achieving an object or information management method wherein, at the request of the customer, the storage company or the like that stores objects or information entrusts the analysis of a stored object or information (for example, an X-ray photograph or X-ray tomographic image data) to a third party.

The invention thus provides analysis service of, among others, data (objects or information) generated in the course of medical treatment and examination or preventive activities. In recent years, with advances in medical technologies, medical practitioners have become more and more specialized. In this context, if a general doctor can easily obtain a medical certificate from an expert doctor having a high analysis skill (for example, the interpreting of X-ray photographs or tomographic image data), it not only serves in the interest of the customer, but also greatly benefits the patient.

The customer can receive analysis service from an experienced doctor just by specifying the data to be analyzed (for example, an X-ray photograph or X-ray tomographic image data) and submitting a request to the storage company to request the analysis by a third party (the customer may specify a particular doctor for the analysis).

Since the storage company already has the requested data in the storage and also has a digitizer for X-ray photographs , etc., the storage company can send the data to the experienced doctor in a short time, and the customer can obtain the result of the analysis (medical certificate) in a short time.

The invention offers the effect of achieving an object or information management method that provides the service in which the storage company pays the analysis fee to the doctor on behalf of the customer, together with the fees for analyses the doctor did for other customers.

The customer, on the other hand, pays the analysis fee to the storage company together with the monthly storage charge. This saves the customer the extra time and labor involved in the payment.

The invention described in claim 18 concerns the object or information management method as described in claim 16 or 17, wherein the method further comprises:
a step for presenting a plurality of analyzing persons to the customer; and
a step for having the customer select at least one analyzing person from among the plurality of analyzing persons, and wherein:
   the first information or the second information is sent to the selected analyzing person.

The invention thus offers the effect of achieving an object or information management method that allows the customer to select a desired doctor from among a plurality of doctors and request the desired doctor for the analysis.

The invention described in claim 19 concerns the object or information management method as described in claim 16 or 17, wherein the method includes, instead of the receiving step and the second sending step, a third sending step for the analyzing person to send the analysis result directly to the customer.

The invention thus offers the effect of achieving an object or information management method that can deliver the analysis result to the customer in a shorter time by allowing the analyzing doctor (for example, the doctor responsible for the analysis service) to send the analysis result directly to the customer.

The invention described in claim 20 concerns the object or information management method as described in claim 16 or 17, wherein the accepting in the accepting step, the sending in the first sending step, second sending step, and third sending step, and the receiving in the receiving step are each performed via a communication line.

The invention thus offers the effect of achieving an object or information management method that can deliver the analysis result to the customer in a shorter time by transmitting information over a communication line.

The invention described in claim 21 concerns the object or information management method as described in claim 16 or 17, wherein
when the customer that sent the object or the second information is denoted as a first customer
the object, the first information, or the second information is an object or information concerning a second customer, and the analysis is carried out at the request of at least either one of the first and second customers.

The invention is particularly effective when the data to be analyzed is data measured on a patient, and when the patient (the second customer) wishes to entrust the analysis of his or her own measurement data (object or the second information) to an experienced doctor outside the hospital where the patient is hospitalized or is being treated as an outpatient.

In the prior art, it has been difficult to fulfill such wishes of the patient, and there has been no other way but to convey the wishes to an experienced doctor through his or her doctor at the hospital.

The invention offers the effect of achieving an object or information management method wherein, at the request of a patient, the storage company or the like that stores objects or information entrusts the analysis of a stored object concerning the patient (for example, an X-ray photograph of the patient) to a third party.

The invention thus provides analysis service of, among others, data (objects or information) generated in the course of medical treatment and examination or preventive activities. In recent years, with advances in medical technologies, medical practitioners have become more and more specialized. In this context, if a patient can easily obtain a medical certificate from an expert doctor having a high analysis skill (for example, the interpreting of X-ray photographs or tomographic image data), the benefit is enormous.

The patient can receive analysis service from an experienced doctor just by specifying the data to be analyzed (for example, an X-ray photograph) and submitting a request to the storage company to request the analysis by a third party (the patient may specify a particular doctor for the analysis).

Since the storage company already has the requested data in the storage and also has a digitizer for X-ray photographs, etc., the storage company can send the data to the experienced doctor in a short time, and the patient can obtain the result of the analysis (medical certificate) in a short time.

The invention thus offers the effect of achieving an object or information management method that provides the service in which the storage company pays the analysis fee to the doctor on behalf of the patient, together with the fees for analyses the doctor did for other customers.

The patient can accomplish the payment just by transferring the money for the analysis fee to the account of the storage company.

The invention described in claim 22 concerns the object or information management method as described in claim 16 or 17, wherein the method further includes:
a step for paying an analysis fee to the analyzing person; and
a step for charging the analysis fee to the customer.

Many difficulties would arise if the hospital as the customer were to pay the fee directly to the doctor who provided the analysis service. Usually, they do not know each other, they are not certain of the other party's credit, and they are not used to issuing slips such as bills.

Further, settling the fee for each analysis involves a great deal of time and labor.

In the present invention, the storage company, on behalf of the customer, pays the analysis fee on a monthly basis to the doctor who provided the analysis service (the storage company contracts with the doctor for the analysis service). The storage company, in turn, charges the analysis fee to the customer together with the monthly storage fee (monthly billing).

The invention thus offers the effect of achieving an object and information management method that provides analysis service the fees for which can be easily settled.

According to the invention described in claim 23, there is provided an object or information management method comprising:
a storing step for storing an object of two-dimensional or three-dimensional shape received from a first customer;
a first storing step for storing in a storage device a first customer identifier for identifying the first customer and a deposit identifier for identifying the object;
an accepting step for accepting an instruction containing therein the first customer identifier, the deposit identifier, and a second customer identifier for identifying a customer other than the first customer, the instruction instructing to grant a request for retrieval of the object if the request is received together with the second customer identifier and the deposit identifier;
a step for comparing the first customer identifier contained in the instruction with the first identifier stored in the storage device, and for rejecting the instruction if the two first customer identifiers are not substantially the same;
an accepting step for accepting the object retrieval request containing therein the second customer identifier and the deposit identifier; and
a step for permitting the retrieval of the object in accordance with the request if the second customer identifier contained in the request substantially matches the second customer identifier contained in the instruction.

When a patient is transferred from hospital B to clinic C, the hospital B reports the past clinical charts to the clinic C, and preferably, hands over the necessary data and documents to the clinic C.

In the prior art, if a doctor at the clinic C wishes to examine a previously taken X-ray photograph or the like, the doctor has had to visit the hospital B and request the retrieval of the X-ray photograph for viewing.

The invention offers the effect of achieving an object or information management method that allows the doctor at the clinic C (corresponding to the second customer) to view on a terminal display at the clinic C the patient's X-ray photograph managed by the hospital B (corresponding to the first customer) whenever the doctor at the clinic C desires.

According to the invention described in claim 24, there is provided an object or information management method comprising:
a storing step for storing an object or two-dimensional or three-dimensional shape received from a first customer, or for storing second information received from a first customer in a storage device, the second information being analog data or digital data;
a first storing step for storing in a storage device a first customer identifier for identifying the first customer and a deposit identifier for identifying the object or the second information;
an accepting step for accepting an instruction containing therein the first customer identifier, the deposit identifier, and a second customer identifier for identifying a customer other than the first customer, the instruction instructing to grant a request for retrieval of the object or the second information if the request is received together with the second customer identifier and the deposit identifier;
a step for comparing the first customer identifier contained in the instruction with the first identifier stored in the storage device, and for rejecting the instruction if the two first customer identifiers are not substantially the same;
an accepting step for accepting the object or second information retrieval request containing therein the second customer identifier and the deposit identifier; and
a step for permitting the retrieval of the object of the second information in accordance with the request if the second customer identifier contained in the request substantially matches the second customer identifier contained in the instruction.

When a patient is transferred from hospital B to clinic C, the hospital B reports the past clinical charts to the clinic C, and preferably, hands over the necessary data and documents to the clinic C.

In the prior art, if a doctor at the clinic C wishes to examine a previously taken X-ray photograph (an object) or X-ray tomographic image data (information), the doctor has had to visit the hospital B and request the retrieval of the X-ray photograph or X-ray tomographic image data for viewing.

The invention offers the effect of achieving an object or information management method that allows the doctor at the clinic C (corresponding to the second customer) to view on a terminal display at the clinic C the patient's X-ray photograph or X-ray tomographic image data managed by the hospital B (corresponding to the first customer) whenever the doctor at the clinic C desires.

According to the invention described in claim 25, there is provided an object or information management method comprising:
a storing step for storing an object of two-dimensional or three-dimensional shape received from a first customer, or for storing second information received from a first customer in a storage device, the second information being analog data or digital data;
a first storing step for storing in a storage device a first customer identifier for identifying the first customer and a deposit identifier for identifying the object or the second information;
an accepting step for accepting an instruction containing therein the first customer identifier, the deposit identifier, and a second customer identifier for identifying a customer other than the first customer, the instruction instructing to transfer a copy of the second information or a copy of first information generated from information contained in the object, to the customer having the second customer identifier;
a step for comparing the first customer identifier contained in the instruction with the first identifier stored in the storage device, and for rejecting the instruction if the two first customer identifiers are not substantially the same;
an accepting step for accepting a request containing therein the second customer identifier and the deposit identifier and requesting storage of the copy of the first information or the second information;
a step for permitting the customer having the second identifier at least to retrieve the first information or the second information for viewing if the second customer identifier contained in the request substantially matches the second customer identifier contained in the instruction; and
a step for charging the customer having the second identifier at least for the storage of the copy if the second customer identifier contained in the request substantially matches the second customer identifier contained in the instruction.

When a patient is transferred from hospital B to clinic C, the hospital B reports the past clinical charts to the clinic C, and preferably, hands over the necessary data and documents to the clinic C.

In the prior art, if a doctor at the clinic C wishes to examine a previously taken X-ray photograph (an object) or X-ray tomographic image data (information), the doctor has had to visit the hospital B and request the retrieval of the X-ray photograph or X-ray tomographic image data for viewing.

The invention offers the effect of achieving an object or information management method wherein a copy of the patient's X-ray photograph or X-ray tomographic image data managed by the hospital B (corresponding to the first customer) is generated and the copy is registered under the name of the clinic C (corresponding to the second customer), thereby enabling the doctor at the clinic C to view on a terminal display at the clinic C the patient's X-ray photograph or X-ray tomographic image data managed by the hospital B whenever the doctor at the clinic C desires.

The invention described in claim 26 concerns the object or information management method as described in any one of claims 23 to 25, wherein
the object or the second information is associated with a customer having a third identifier, and
the first customer identifier is replaced by the third identifier.

When a patient is transferred from hospital B (corresponding to the first customer) to clinic C (corresponding to the second customer), the hospital B reports the past clinical charts to the clinic C, and preferably, hands over the necessary data and documents to the clinic C.

In the prior art, if the patient wishes a doctor at the clinic C to examine a previously taken X-ray photograph (an object) or X-ray tomographic image data (information), the patient has had to ask the doctor at the clinic C and the doctor has had to visit the hospital B and request the retrieval of the X-ray photograph or X-ray tomographic image data for viewing.

The invention offers the effect of achieving an object or information management method that allows the doctor at the clinic C to view on a terminal display at the clinic C the patient's X-ray photograph or X-ray tomographic image data managed by the hospital B whenever requested by the patient (corresponding to the customer having the third identifier).

The novel features of the invention will be hereinafter fully described and particularly pointed out in the appended claims, and the configuration and details of the invention, together with other objects and features thereof, will become better understood and appreciated by reference to the following detailed description when considered in conjunction with the accompanying drawings.

### BRIEF DESCRIPTION OF THE DRAWINGS

Figure 1 is a diagram schematically illustrating the configuration of a first embodiment of the present invention.

Figure 2 is a diagram schematically illustrating the configuration of a second embodiment of the present invention.

Figure 3 is a diagram schematically illustrating the configuration of a third embodiment of the present invention.

Figure 4 is a diagram showing a storage list according to an embodiment of the present invention.

Figure 5 is a diagram showing a storage list according to an alternative of the present invention.

Figure 6 is a diagram showing a billing list according to an embodiment of the present invention.

Figure 7 is a diagram showing an example of a chest X-ray photograph stored in an object warehouse.

Figure 8 is a diagram schematically illustrating the configuration of a fourth embodiment of the present invention.

Figure 9 is a diagram schematically illustrating the configuration of a fifth embodiment of the present invention.

Figure 10 is a diagram for explaining an object management method according to the prior art.

Figure 11 is a diagram for explaining an information management method according to the prior art.

Figure 12 is a flow chart for the first embodiment of the present invention.

Figure 13 is a flow chart for the fifth embodiment of the present invention.

It will be recognized that some or all of the Figures are schematic representations for purposes of illustration and do not necessarily depict the actual relative sizes or locations of the elements shown.

### DETAILED DESCRIPTION OF THE INVENTION

Embodiments illustrating the best mode for carrying out the invention will be described below with reference to the accompanying drawings.

### <<Embodiment 1>>

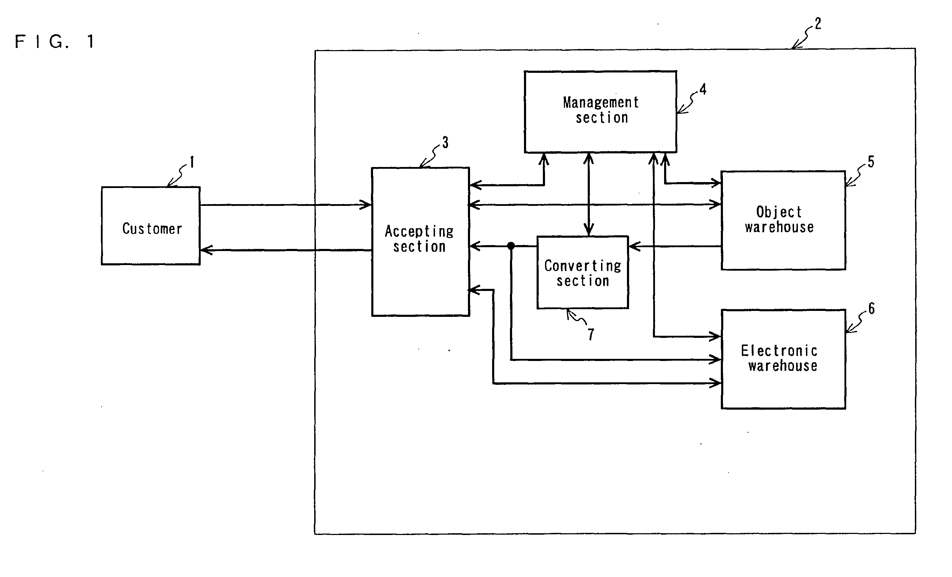

A first embodiment of the present invention will be described below with reference to Figure 1.

### <Description of the General Configuration of the First Embodiment (Figure 1)>

Figure 1 illustrates schematically the configuration of the first embodiment of the present invention.

Figure 1 shows a storage system 2 which stores various objects and information owned by medical institutions such as hospitals and clinics.

Customer 1 (for example, a hospital) entrusts the storage of an object or information to the storage system (for example, a storage company) 2 by specifying at least the customer's name and the object (or information) to be stored.

The "object" includes, for example, an X-ray photograph, a clinical chart written on paper, etc.

The "information" includes, for example, image data such as photographed image data, audio data such as the sound of heartbeats, graphs such as electrocardiograms, X-ray tomographic image data, or data such as electronic clinical charts.

The "information" also includes information consisting of a data sequence as an intangible transmitted to the storage system 2 via the Internet, and information recorded on a recording medium, for example, a DVD (though this is physically an object).

The storage system of this embodiment mainly stores such objects and information, that is, data produced in the course of medical examination and treatment, but can also store other items such as past accounting slips, etc. from hospitals.

Generally, when the customer entrusts the storage of information, information generated by a diagnostic instrument such as a CT scanner or an image recorded on an electronic medium such as a DVD is transmitted to a terminal such as a computer via a LAN or read into a computer by playing it back on a DVD player connected to the computer, and the information or image data is transmitted from the computer to the storage system.

However, if the diagnostic instrument has an intelligent communication function and is assigned a customer ID, the diagnostic instrument may by itself communicate with the storage system and transmit the information (image data, etc.) output from it directly to the storage system.

Likewise, if the electronic media playback apparatus such as a DVD player has an intelligent communication function and is assigned a customer ID, the playback apparatus may by itself communicate with the storage system and transmit the information output from it (DVD playback output, etc.) directly to the storage system.

The request for storage can be made in various ways; for example, the customer may deliver a letter of request in person or may mail it, or otherwise may prepare a letter of request on a computer at the customer and transmit it via the Internet to an accepting section 3 in the storage system.

Generally, information consisting of data sequences is transmitted from the customer 1 to the accepting section 3 in the storage system 2 via a communication line (for example, the Internet).

Objects and recording media are generally sent via mail from the customer 1 to the accepting section 3 in the storage system 2.

The accepting section 3 accepts the request for storage by assigning the customer a unique identifier (customer ID - customer identifier) specified by a management section 4 and also assigning the object or information to be stored a unique identifier (deposit ID - deposit identifier) specified by the management section 4. In the case of a storage system that manages medical information such as X-ray photographs, preferably a unique identifier (patient ID - patient identifier, specified by the management section 4) is also assigned to the patient related with the object (for example, an X-ray photograph) or information (for example, an electronic clinical chart) to be stored.

When an object (for example, an X-ray photograph) is received for entrustment, the accepting section 3 stores the object in an object warehouse 5; on the other hand, when information (for example, a data sequence or a recording medium such as a DVD) is received for entrustment, the accepting section 3 stores the information in an electronic warehouse 6.

The "object warehouse" is a warehouse for storing ordinary objects.

The "electronic warehouse" is a warehouse for storing intangible information (data) by recording it on a recording medium such as a hard disk or an optical disk.

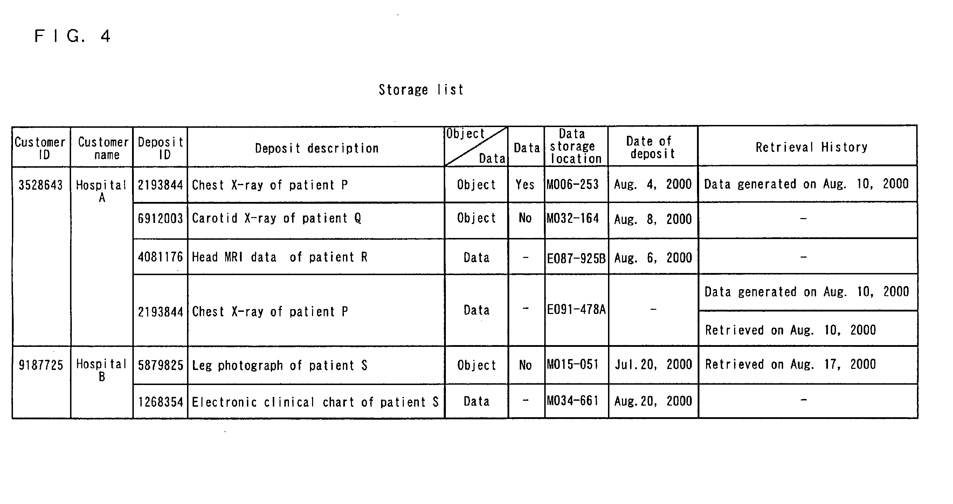

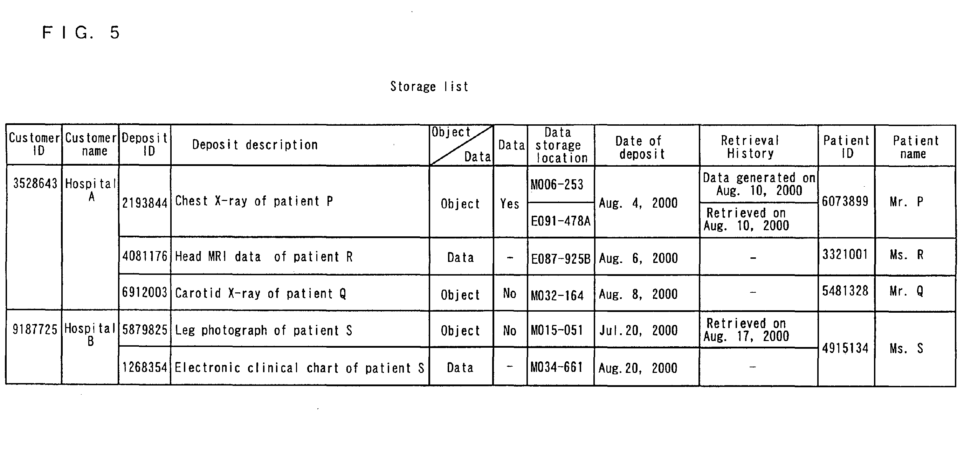

The management section 4 records the date of acceptance, customer identifier, object (or information) identifier, storage place information, patient identifier, etc. (see Figure 4 or 5) together with the fact that the object or information has been stored in the object warehouse or electronic warehouse.

The object warehouse 5 and the electronic warehouse 6 are separate entities, but the management section 4 manages both warehouses in consolidating fashion.

For example, suppose that a hospital B as the customer 1 entrusted the storage of a photograph (object) of an affected site (leg) of a patient S suffering burns and the electronic clinical chart (information) of the patient S. In this case, the photograph of the affected site of the patient S is stored in the object warehouse, and the electronic clinical chart of the patient S is stored in the electronic warehouse 6. Here, since the management section 4 manages data such as X-ray photographs and electronic clinical charts associated with the hospital B or the patient S in consolidating fashion, a consolidated management list such as shown in Figure 4 or 5 can be displayed on a display unit (a terminal display, etc.).

The customer 1 can request the retrieval of the stored object (or information) by sending to the storage system 2 the customer identifier (for example, 3528643 for hospital A in Figure 4), the identifier of the requested object (or information) (for example, 2193844 for the X-ray photograph of patient P in Figure 4), and a letter of request requesting the retrieval of the object (or information).

The accepting section 3 in the storage system 2 identifies the identifiers attached to the request against the customer ID and deposit ID carried in the storage list (Figure 4 or 5), and accepts the request if they match.

The accepting section 3 passes the customer ID and the deposit ID of the requested object or information to the management section 4. The management section 4 records and stores these pieces of information on a recording device (not shown).

The management section 4 searches the storage list (Figure 4 or 5) for the deposit ID that matches the received deposit ID.

If a matching deposit ID is found, then if the deposited article is an object the management section 4 reports the storage place of the object to the object warehouse 5 and directs it to retrieve the object. If the deposited article is information, the management section 4 reports the storage place of the information to the electronic warehouse 6 and directs it to retrieve the information.

As will be described later, even in the case of a retrieval request for a stored object, if that object is stored in the form of electronic data in the electronic warehouse 6, the management section 4 directs the electronic warehouse 6 to retrieve the electronic data.

If an object is retrieved from the object warehouse 5, the object is converted by a converting section 7 into electronic data (except when the customer explicitly requests the delivery of the object itself).

In the first embodiment, since the storage system 2 mainly stores medical data such as X-ray photographs, what the customer wants is, in most cases, the retrieval of the information contained in the requested object (for example, the retrieval of an X-ray photograph for viewing), unlike the case of an ordinary object storage warehouse (which stores, for example, disused office desks). Therefore, rather than sending the X-ray photograph to the customer by mail or other means, generating electronic data (bit mapped image data) of the X-ray photograph and sending it directly to the customer via the Internet would better meet the needs of the customer. Moreover, sending the electronic data, rather than the X-ray photograph itself, can save time and cost.

Accordingly, whether the customer requests the retrieval of an object or the retrieval of information, information is retrieved (or generated), and the retrieved (or generated) information is passed to the accepting section 3 (except when the customer requests the delivery of the actual object).

The accepting section 3 transmits the information to the customer 1 via a leased line or the Internet 26.

The management section 4 calculates monthly storage charges (determined based on the number of stored objects and information items) starting from the date of acceptance and handling charges for the delivery of objects or information to the customer (determined based on the number of retrieved objects or information items and the number of retrievals), and charges them to the customer on a monthly basis (billing).

As described above, in the first embodiment, only the customer who entrusted the storage of an object or information is allowed to access the stored object or information.

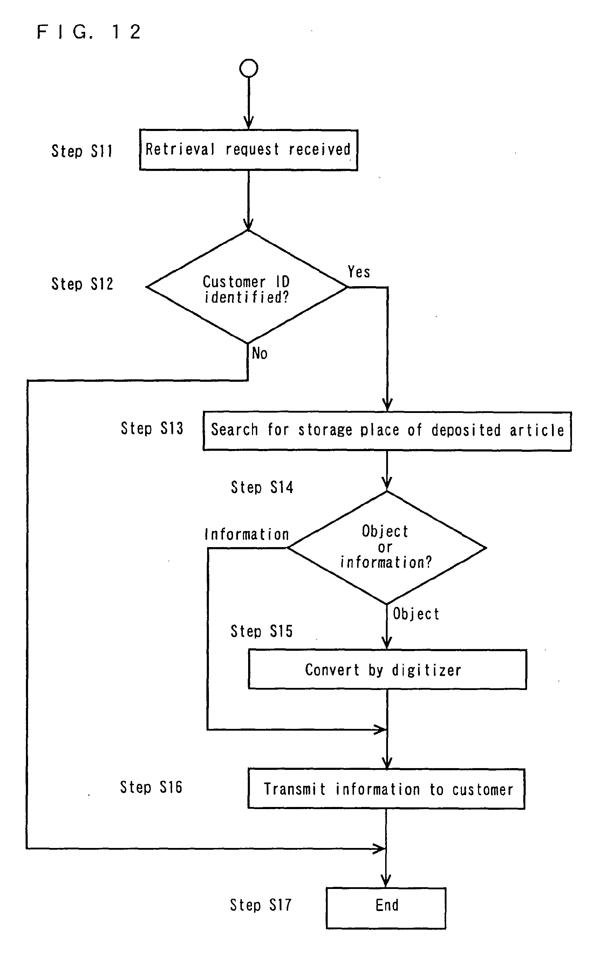

### <Explanation of Flow Chart for the First Embodiment (Figure 12)>

Figure 12 is a flow chart for the first embodiment.

When a request for retrieval of an object or information is received from the customer, the request is processed in accordance with the procedure shown in Figure 12.

First, a request for retrieval of an object or information is received from the customer (step S11).

Next, the customer ID contained in the received data is identified. A search is made through the storage list (Figure 4 or 5), and if the same customer ID is found, the retrieval request is granted; on the other hand, if the same customer ID is not found, the retrieval request is rejected (step S12).

When the request is granted, a search is made for the storage place of the deposited article (object or information) that matches the deposit ID contained in the retrieval request (step S13).

If the deposited article is information, the process proceeds to step S16, while if the deposited article is an object, the process proceeds to S15 (step S14).

If the deposited article is an object, the information contained in the object is converted into first information by using a digitizer (step S15). Typically, an X-ray photograph is converted into bit mapped image data.

The information is transmitted to the customer (step S16).

### <<Embodiment 2>>

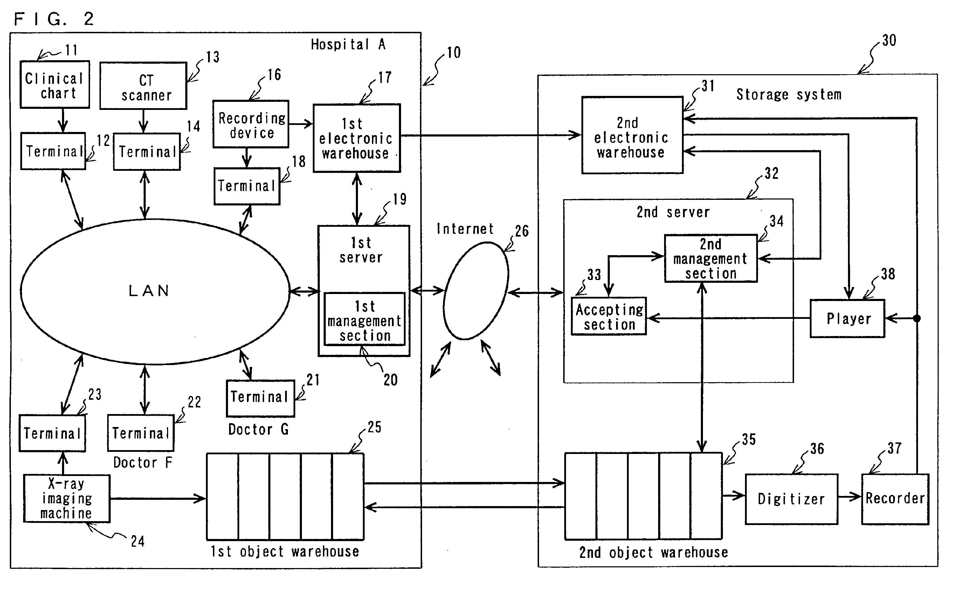

A second embodiment of the present invention will be described below with reference to Figure 2.

### <Explanation of the General Configuration of the Second Embodiment (Figure 2)>

The storage system of the second embodiment has a first storage place for objects and information within the customer premises, in this case, a hospital, and has a second storage place, that is, a large storage place for objects and information, at a location remote from the hospital.

The first storage place is a temporary storage place having a limited storage capacity, but allows the customer to quickly retrieve the stored objects or information.

The second storage place is a permanent storage place having a large storage capacity. Although inferior to the first storage place in terms of accessibility, the second storage place allows the customer to easily access the stored objects (or information) via a leased line or via the Internet.

Hospital A as the customer, indicated at 10, has various terminals connected to a LAN. The LAN is managed by a first server 19.

An electronic clinical chart 11 entered by a doctor into a terminal 12 is sent via the LAN and a terminal 18 to a recording device 16. The recording device 16 is, for example, a DVD recorder. The electronic clinical chart is recorded on a DVD. At the same time, a first management section 20 in the first server 19, notified of the recording of the electronic clinical chart on the DVD, specifies the storage location of the DVD on which the electronic clinical chart has been recorded.

The DVD with the electronic clinical chart recorded thereon is stored at the specified storage location in the first electronic warehouse 17.

A CT scanner 13 generates computer processed X-ray tomographic image data of a patient. The X-ray tomographic image data is transmitted to the recording device 16 via a terminal 14, the LAN, and the terminal 18 in this order. The X-ray tomographic image data is recorded on a DVD. At the same time, the first management section 20 in the first server 19, notified of the recording of the X-ray tomographic image data on the DVD, specifies the storage location of the DVD on which the X-ray tomographic image data has been recorded.

The DVD with the X-ray tomographic image data recorded thereon is stored at the specified storage location in the first electronic warehouse 17.

An X-ray imaging machine 24 produces X-ray photographs. When an X-ray photograph is taken, the doctor enters notification into a terminal 23. Upon receiving the notification that an X-ray photograph has been taken, the first management section 20 in the first server 19 specifies the storage location in the first object warehouse 25 at which the X-ray photograph is to be stored.

The X-ray photograph is stored at the specified storage location in the first object warehouse 25.

Doctors F and G working at the hospital A can access the objects and information stored in the first and second storage places as desired through their terminals 22 and 21.

For example, the doctor G can retrieve a list of information, etc. associated with the hospital A from the storage system 30 and have it displayed on the screen of his terminal (see the hospital A column in the storage list shown in Figure 4 or 5).

When transmitting information (sender information) from the storage system 30 to the customer, mutual authentication must be established at all times. In the present embodiment, mutual authentication is performed using a password. Any appropriate method of mutual authentication may be employed; for example, fingerprints, handwritten signatures, etc. can be used. By mutual authentication, leakage of customer secrecy is prevented.

In the case of a large customer such as the hospital A, a personal identifier for identifying each responsible doctor such as doctor F, doctor G, etc. or a terminal equipment identifier for identifying each terminal such as terminal 21, 22, etc. is assigned under the customer name and customer ID of the hospital A.

Accordingly, when a retrieval request, etc. are received from the hospital A, the accepting section 33 in the storage system 30 checks only the customer ID of the hospital A in the customer identifiers contained in the retrieval request to see whether that customer ID matches the customer ID of the hospital A carried in the storage list, and can disregard the doctor's personal ID that follows the customer ID of the hospital A. In this way, every doctor working at the hospital A and having a personal ID can freely access any object or information owned by the hospital A.

The storage list contains only the customer ID of the hospital A (personal IDs are not contained), but provision may be made for the accepting section 33 to identify not only the customer ID of the hospital A but also the personal ID when identifying the customer, thereby allowing only a limited number of authorized doctors at the hospital A to access the stored objects or information.

In the example of Figure 2, anyone can freely access the objects or information stored in the first storage place (within the hospital A) (identification of personal IDs is not done here).

However, in the first storage place also, provision may be made for the first management section 20 to identify the personal ID when a request for access to an object or information is received. That is, a doctor wishing to access an object or information sends his or her own personal ID to the first management section 20. The first management section 20 examines its internal listing of authorized doctors, and searches for the personal ID that matches the received personal ID. If a matching personal ID is found, the first management section 20 permits the doctor to retrieve the object or information.

The administrator responsible for the first electronic warehouse 17 and first object warehouse 25 in the first storage place may be the storage company operating the storage system 30, or may be the customer.

When the administrator responsible for the first electronic warehouse 17 and first object warehouse 25 is the storage company, usually the storage charge is calculated starting from the time that an object or information is first stored in the first electronic warehouse 17 or the first object warehouse 25 in the first storage place.

On the other hand, when the administrator responsible for the first electronic warehouse 17 and first object warehouse 25 is the customer, usually the storage charge is calculated starting from the time that an object or information is first stored in the second electronic warehouse 31 or the second object warehouse 35 in the second storage place.

Of course, the time at which the calculation of the storage charge starts can be chosen freely.

The object (for example, an X-ray photograph) or information (for example, X-ray tomographic image data) stored in the first storage place is transferred to the second storage place when a predetermined period has elapsed.

This predetermined period can be specified for each customer, or the customer can specify it for each object such as an X-ray photograph or for each information item such as an electronic clinical chart.

Alternatively, each time the customer requests, the objects or information that have been stored in the first storage place up to that time may be collected and stored in the second warehouse, without providing any specific period.

Information can be transmitted to the second electronic warehouse 31 in the second storage place via the Internet 26, but objects such as X-ray photographs and recording media such as DVDs need to be sent by mail or other delivery means. The customer can send such objects and recording media directly to the storage system 30, but as an alternative method, personnel from the storage system 30 may periodically visit the customer to collect the objects, etc. that have been stored for more than a predetermined period of time.

The storage system 30 is located in the second storage place. The second storage place is usually far from the customer 10, that is, the hospital A, but has a sufficiently large space, and the second object warehouse 35 and the second electronic warehouse 31 both have a sufficiently large storage capacity.

When a recording medium such as a DVD is shipped out from the first electronic warehouse 17, the first management section 20 transmits the shipping information to the second server 32 in the storage system 30. The accepting section 33 in the second server 32 passes the received shipping information to the second management section 34. The second management section 34 specifies the storage location in the second electronic warehouse 31 at which the recording medium shipped by mail is to be stored.

The recording medium received via mail is stored at the specified location in the second electronic warehouse 31.

Likewise, when an object such as an X-ray photograph is shipped out from the first object warehouse 25, the first management section 20 transmits the shipping information to the second server 32 in the storage system 30. The accepting section 33 in the second server 32 passes the received shipping information to the second management section 34. The second management section 34 specifies the storage location in the second object warehouse 35 at which the object shipped by mail is to be stored.

The object received via mail is stored at the specified location in the second object warehouse 35.

For example, the doctor F at the hospital A transmits the customer ID 3528642 of the hospital A with his personal ID attached to it, the deposit ID 6912003 of the object (X-ray photograph of a carotid artery of a patient Q) he wishes to retrieve for viewing, and a letter of retrieval request, via his terminal 22 onto the Internet 26 for transmission to the storage system 30.

The accepting section 33 searches the storage list (Figure 4 or 5) for the customer ID that matches the received customer ID. If a matching customer ID is found, the letter of request received from the doctor F is passed to the second management section 34.

The second management section 34 searches the storage list (Figure 4 or 5) for the deposit ID that matches the received deposit ID 6912003. If the record indicating "CAROTID X-RAY OF PATIENT Q" that the doctor wishes to retrieve is found, the second management section 34 directs the second object warehouse 35 to retrieve the X-ray photograph stored at the storage location M032-164.

The manager of the second warehouse retrieves the X-ray photograph of the carotid artery of the patient Q from the specified storage location, and converts the X-ray photograph into electronic data (bit mapped image data) by using a digitizer 36 (which corresponds to the converting section 7 in Figure 1).

When the digitizing is finished, the X-ray photograph is stored again at the original storage location.

Next, the obtained bit mapped image data is sent to a recorder 37 (for example, a DVD recorder) for recording on a DVD. The recorded DVD is played back on a player 38 (in the present embodiment, a DVD player). The reproduced image is sent to the accepting section 33. Under instruction from the second management section 34, the accepting section 33 transmits the reproduced image to the doctor F's terminal 22 at the hospital A.

In the present embodiment, the recorder 37 is constructed from a DVD recorder or a hard disk, depending on the needs of the customer. The recorder 37 may be constructed from both a DVD recorder and a hard disk.

Preferably, the image is transmitted in compressed and encrypted form. Any appropriate compression scheme can be chosen, such as EPSE, GIF, JPEG, PICT, or TIFF. For encryption, DES, RSA, FEAL, or other suitable scheme can be employed.

In the present embodiment, the customer and the storage system have the same flash memory cards, and numbers recorded on the respective flash memory cards are used for encryption at the transmitting end and for decryption at the receiving end. Since the number recorded on the flash memory card is different from one customer to another, secrecy of each customer can be ensured.

The DVD recorded by the recorder 37 is stored in the second electronic warehouse at the storage location specified by the second management section 34.

At the hospital A as the customer, when a chest X-ray of patient P is taken by the X-ray imaging machine 24, the X-ray photograph is stored in the first object warehouse for the time being.

When a prescribed period has elapsed, the X-ray photograph is transferred from the first storage place (the first object warehouse) to the second storage place.

Likewise, when an X-ray tomographic image of patient V is taken by the CT scanner 13, the tomographic image data is stored in the first electronic warehouse for the time being.

When a prescribed period has elapsed, the image data is transferred from the first storage place (the first electronic warehouse) to the second storage place.

The first storage place provides only a limited storage capacity, but the customer can immediately access the stored objects or information.

The second storage place is located far from the customer, but has a sufficiently large storage space and can store objects or information as long as the customer desires.

Further, though the second storage place is located far from the customer, the customer can easily retrieve the stored objects or information for viewing by using the Internet 26.

Suppose, for example, that the doctor F who is seeing the patient Q wants to compare the recently taken carotid X-ray photograph of the patient Q (which the doctor F keeps at hand) with the carotid X-ray photograph of the patient Q taken half a year ago (stored in the second object warehouse 35). In this case, the doctor F requests the storage system 30 via the Internet to retrieve the latter photograph. After a short while, the carotid X-ray photograph of the patient Q taken half a year ago is displayed in the form of bit mapped image data on the display screen of the terminal 22. In this way, even when an object or information is stored in the second storage place, the doctor F can call up the image to the display in the presence of the patient and explain how the treatment has progressed for the past half year.

In the prior art, once an X-ray photograph as an objet was stored in a storage warehouse, the X-ray photograph was not delivered immediately upon request, but was delivered on the next day at the earliest.

By contrast, in the present embodiment, when the storage of an object is entrusted to the storage system 30, the customer can easily and quickly access the stored object or information even when the object is stored in the second storage place, because the storage system 30 can digitize the information contained in the object and transmit the digitized data to the customer via the Internet.

It is known empirically that when data, etc. are measured on a patient, the data, etc. are frequently used shortly after the measurement, but the probability of use or the number of times that the data, etc. are used decreases rapidly as the time elapses.

In view of this, the data, etc. are stored in the first storage place within the hospital A for some time after the measurement of the data, etc. though the storage capacity of the first storage place is not large. During that period, the doctor at the hospital A can immediately retrieve the data any time, or can diagnose using the actual X-ray photograph.

When a prescribed period has elapsed, the data, etc. are transferred from the first storage place to the second storage place, thus freeing the space in the first storage place for storing another object or information.

It takes more time to retrieve the data, etc. once they are stored in the second storage place than when they were stored in the first storage place, but the probability that the need to retrieve the data, etc. will arise is low, as described above, and if the need to retrieve the data, etc. does arise, they can be accessed easily and at high speed.

The second management section 34 calculate storage charges based on the number of objects or information items entrusted for storage, the number of retrievals requested, etc. , and bills the customer hospital A on a monthly basis.

When storage charges also accrue from the first storage place, the second management section 34 periodically communicates with the first management section 20 to keep track of the storage state in the first storage place, since the first management section 20 manages the storage and usage states of the first storage place.

The second management section 34 calculates the storage charges accruing from the first storage place (managed by the first management section 20) as well as the storage charges accruing from the second storage place (managed by the second management section 34), and changes the total sum to the hospital A.

In an alternative embodiment, the first management section 20 is omitted. The second management section 34 manages not only the storage state in the second storage place, but also the storage state in the first storage place via the Internet 26. In this case, the first server 19 acts like a terminal subservient to the second management section 34.

### <<Embodiment 3>>

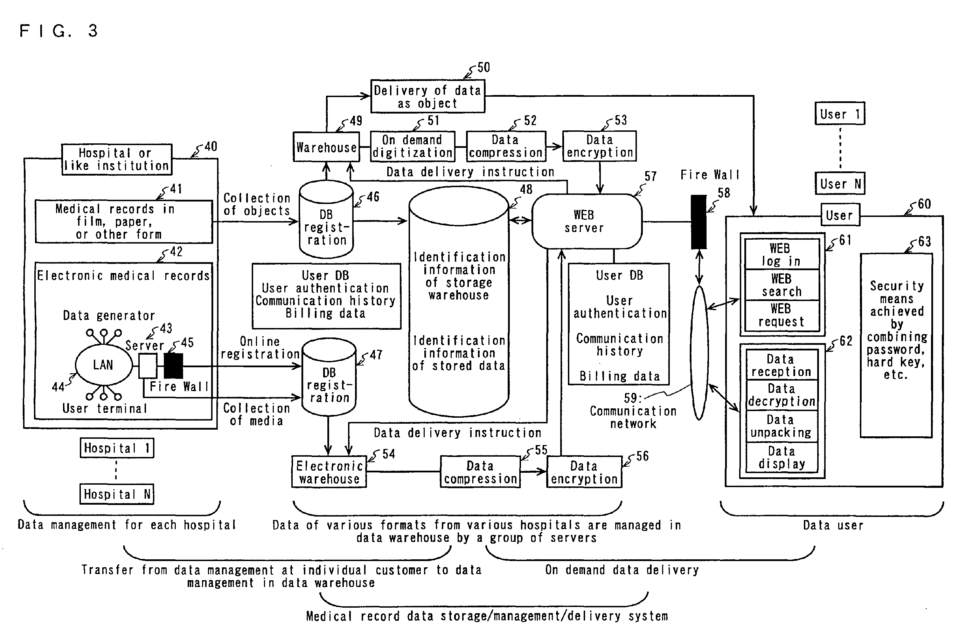

A third embodiment of the present invention will be described below with reference to Figure 3.

### <Explanation of Storage System with Ensured Security (Figure 3)>

Figure 3 illustrates a storage system with ensured security.

Customer 40 includes a hospital, etc. User 60 at the right in the figure is also a customer. Objects and information are shown as flowing from the left to the right.

The customer 40 entrusts the storage of objects or information to the storage system (which is a medical record data storage/management/delivery system shown in the center).

Objects and information entrusted for storage include films such as X-ray photographs, clinical charts 41 written on paper or the like (objects), and electronic clinical charts 42 (information).

The electronic clinical charts 42 are stored into a server 43 for centralized management, for example, via an intra-hospital LAN 44. If an unauthorized person outside the hospital attempts to access the LAN 44, the access is denied by the presence of a firewall 45.

A DVD recorder is connected to the server 43 to record information on a DVD as it is collected.

When storing the DVD in an electronic warehouse, the information may be encrypted or may not be encrypted. Preferably, the information is encrypted to render the contents unintelligible to other than authorized persons in the hospital. Any appropriate encryption scheme can be used.

Each DVD (medium) has its unique ID recorded thereon during the course of manufacturing. Different IDs are recorded on different media.

The present embodiment achieves reliable security at low cost by using the unique ID of each DVD (medium).

When recording by the DVD recorder, the unique ID of the DVD medium is read out using the playback function of the DVD recorder. The information to be recorded is encrypted using the thus read out unique ID of the DVD medium. When reproducing the information recorded on the DVD, the unique ID of the DVD medium is read out using the playback function of the DVD recorder. The information is decrypted using the thus read out unique ID of the DVD medium.

DVD units installed in a hospital are, in most cases, DVD players (playback-only machines). Each DVD player is configured so as not to be able to read the unique ID of any DVD medium. The encrypted information recorded on the DVD medium, therefore, cannot be decrypted by any of these DVD players. Security within the hospital can thus be ensured.

Here, since the DVD player in the storage system must have the capability to read the unique ID of each DVD medium, in practice a DVD recorder having a playback function is used.

Medical records such as films as objects are periodically collected (for example, twice a month) and transferred to the storage system. The collected medical records as objects are registered with a data base registration section 46, and the objects are stored in an object warehouse 49.

The data base registration section 46 creates an object storage list for each user, and stores it in a data base 48. As will be described later, since the storage list is created using commercially available data base software such as ACCESS, it is possible to create a customized storage list from the data base by extracting records in particular fields. The created storage list is stored in the data base 48.

The data base registration section 46 also authenticates the customer who entrusted the storage of objects (user authentication) by referring to the customer ID list stored in the data base 48.

The data base registration section 46 keeps track of the storage state and retrieval state of the objects entrusted from each customer, and manages customer communication history and billing data. The created communication history, billing data, etc. are stored in the data base 48.

The data base registration section 46 registers the storage location of each object stored in the object warehouse 49, and stores the storage location data in the data base 48.

Recording media such as DVDs holding electronic medical records stored thereon are periodically collected (for example, twice a month) and transferred to the storage system. The collected DVDs are registered with a data base registration section 47, and the DVDs are stored in an electronic warehouse 54.

The data base registration section 47 creates an object storage list for each user. A customized storage list can be created from the data base by extracting records in particular fields. The created storage list is stored in the data base 48.

The data base registration section 47 also authenticates the customer who entrusted the storage of information such as DVDs (user authentication) by referring to the customer ID list stored in the data base 48.

The data base registration section 47 keeps track of the storage state and retrieval state of the DVDs, etc. entrusted from each customer, and manages customer communication history and billing data. The created communication history, billing data, etc. are stored in the data base 48.

The data base registration section 47 registers the storage location of each DVD stored in the electronic warehouse 54, and stores the storage location data in the data base 48.

The customer 40 can perform online registration for the storage of recording media such as DVDs by communicating through the server 43 with the data base registration section 47 via the Internet.

As described above, when registering the storage, whether it be an object or information (or a recording medium), all the data are stored in the data base 48. In the data base 48, the registration data, communication history data (including retrieval records, etc.), billing data, etc. are consolidated between the objects and the information.

Accordingly, though the storage of objects and the storage of information are registered by the different data base registration sections 46 and 47, the customer can call up a consolidated storage list of objects and information for display on the terminal screen at the customer. For example, from the storage list shown in Figure 4 or 5, only the records associated with the hospital A can be displayed on the terminal screen at the hospital A.

Further, the storage system bills the hospital A by producing a bill stating the total sum of the object storage charges and the information storage charges, rather than producing separate bills for the object storage charges and the information storage charges.

The user (customer) 60 can retrieve the entrusted objects or information for viewing via a communication network such as the WEB.

The user logs in to the WEB and searches the WEB for the site of the storage system. The user transmits a retrieval request, together with his or her customer ID and the deposit ID of the deposited article he or she wishes to retrieve for viewing, to the storage system (WEB request) (61).

The WEB server 57 in the storage system searches the customer ID list stored in the data base 48 for the customer ID that matches the received customer ID. If a matching customer ID is found, the storage system grants the user request, and if there is no matching customer ID, rejects the user request.

By combining a password, hard key, etc., greatly enhanced security can be achieved (63).

If a third party attempts to intrude to access the data base in the storage system, the intrusion is prevented by the firewall 58.

When the user requests the retrieval of a stored object for viewing, the WEB server 57 searches the object warehouse 49 and retrieves the requested object. If the requested object is, for example, an X-ray photograph, the X-ray photograph is converted into digital video data by using a digitizer (on demand digitization 51). The obtained digital video data is compressed by using a compression scheme such as GIF (data compression 52). The compressed data is encrypted by using DES or other encryption scheme (data encryption 53). The WEB server 57 transmits the thus compressed and encrypted data to the user via the communication network 59. The user receives the data, decrypts and unpacks the data, and displays it on the display screen of the user's personal computer (62).

When the user requests the retrieval of stored information (recorded on a recording medium) for viewing, the WEB server 57 searches the electronic warehouse 54 and retrieves the requested recording medium. The retrieved recording medium is played back on a player (for example, a DVD player) to reproduce the digital video data recorded thereon. The obtained digital video data is compressed by using a compression scheme such as GIF (data compression 55). The compressed data is encrypted by using DES or other encryption scheme (data encryption 56). The WEB server 57 transmits the thus compressed and encrypted data to the user via the communication network 59. The user receives the data, decrypts and unpacks the data, and displays it on the display screen of the user's personal computer (62).

When the user requests the retrieval and delivery of the stored object itself, the WEB server 57 searches the object warehouse 49 and retrieves the requested object. The requested object thus retrieved is then delivered to the user via courier service (delivery of data as object 50).

The user receives the object.

### <Explanation of the Storage List (Figures 4 and 5)>

Figures 4 and 5 each show the storage list stored in the management section 4 in the present embodiment.

The objects and information entrusted for storage are entered in the list in a manner consolidating the objects and information as shown in Figure 4 or 5 (rather than entering the objects and information in separate lists).

Figures 4 and 5 explicitly illustrate how the data are linked together according to the present embodiment.

In Figures 4 and 5, the lists are organized by customer name (or customer ID). In the present embodiment, the storage list is created using commercially available data base software (for example, Microsoft Access) that runs on Windows. Accordingly, the storage list can be displayed based on a particular field. For example, if the storage list is displayed by organizing the data by patient ID (Figure 5), the data concerning patient P are shown grouped together (including the case in which the patient P is consulting doctors at different hospitals or clinics).

In Figure 4 (and Figure 5), all objects and information that the customer hospital A has entrusted for storage are shown grouped together. Every customer has a customer ID which is used to identify the customer and prevent a third party's intrusion into the data base. The storage list can also contain the password of each customer in addition to the customer ID. When the customer requests the retrieval of a stored object, etc., the customer is required to enter his or her password in addition to the customer ID; this prevents a third party from accessing the data base.

The customer name field, the deposit description field, etc. carry the name of the customer, description of the deposited article, etc. which are useful when displaying the storage list on the screen.

Every deposited article has a unique ID of its own.

Each deposited article is stored in the object warehouse 5 or the electronic warehouse 6. The object/data field indicates whether the deposited article is an object or information (data).

The data field, which is valid when the deposited article is an object, indicates whether the object has been converted by the digitizer (converting section 7) into electronic data (bit mapped image data) for storage. The indication "YES" means that not only is the object itself stored in the object warehouse 5, but also its electronic data is stored in the electronic warehouse 6.

Storage location indicates the location in the object warehouse 5 or the electronic warehouse 6 at which the deposited article is stored. For example, the chest X-ray photograph of the patient P is stored at location 253 on rack M006 (M006-253) in the object warehouse 5. The head MRI data of the patient R is recorded on a DVD stored at location 925 on rack E087 in the electronic warehouse 6. Generally, more than one piece of data is recorded on one DVD, and the head MRI data of the patient R is recorded at location B on the DVD (E087-925B).

Date of deposit indicates the starting date of storage. Retrieval history not only serves as data based on which the storage system 2 bills the customer, but also provide convenience to the hospital A when it investigates the past history of use of the stored data.

In Figure 4, the chest X-ray photograph of the patient P and its electronic data are registered as different items but assigned the same deposit ID 2193844. In the calculation of storage charges, the chest X-ray photograph of the patient P and its electronic data are treated as the same article, while in the management of the object warehouse 5 and electronic warehouse 6, they are treated as different articles.

Here, the chest X-ray photograph of the patient P and its electronic data may be assigned different deposit IDs.

Within the storage system 2 or 30, the list shown in Figure 4 or 5 can be displayed in its entirety. On the other hand, at the hospital A as a customer, a list consisting only of the records of the objects and information that the hospital A has entrusted for storage can be displayed for viewing.

In the storage list of Figure 5, the chest X-ray photograph of the patient P and its electronic data are registered as a single item.

Further, the storage list of Figure 5 has a patient ID field and a patient name field. When a patient requests the retrieval of his or her own chest X-ray photograph, these fields are used to identify the patient and to bill the patient at a later date, as will be described later. Likewise, when it becomes necessary to transfer all the deposited articles concerning the patient P from hospital A to hospital D because the patient P is transferred from hospital A to hospital D, a storage list can be created by extracting the records of the patient P, and the customer name and customer ID of the hospital A for all the deposited articles concerning the patient P can be converted at once to the customer name and customer ID of the hospital D.

When a request for the retrieval of the chest X-ray photograph of the patient P is received from the hospital A, the storage system 2 retrieves the chest X-ray photograph of the patient P from the object warehouse 5, converts it into electronic data by means of the conversion section 7 (digitizer), and transmits the electronic data to the hospital A.

Since creating the electronic data by the conversion section 7 is a time and labor consuming job, it is desirable that the generated electronic data be also stored as long as the chest X-ray photograph of the patient P is stored. Once the electronic data is stored, the next time the hospital A requests the retrieval of the chest X-ray photograph of the patient P (since the hospital A entrusted the storage of the chest X-ray photograph of the patient P as a physical object, the hospital A requests the retrieval of the chest X-ray photograph of the patient P, not its electronic data), the electronic data representing the chest X-ray photograph of the patient P can be immediately transmitted to the hospital A by retrieving it from the electronic warehouse 6.

The procedure for checking the presence or absence of electronic data will be described in detail below.

When the hospital A transmits a letter of retrieval request for the chest X-ray photograph of the patient P, together with its customer ID 3528643 and deposit ID 2193844, to the storage system 2, the accepting section 3 passes the letter of request to the management section 4 after verifying that the received customer ID matches the customer ID carried in the storage list.

The management section 4, referring to the storage list of Figure 4, searches the information stored in the electronic warehouse 6 for the deposit ID 2193844 at first. If the same deposit ID is found, the management section 4 directs the electronic warehouse 6 to retrieve the electronic data from the storage location E091-478A. The retrieved electronic data is transmitted to the hospital A.

In this search method, even when the customer requests the retrieval of a stored object, the management section 4 first searches the information stored in the electronic warehouse 6; therefore, if both the requested object and its electronic data are stored, the electronic data is preferentially selected. It is only when the electronic data having the same deposit ID does not exist that the search is made for the requested object.

Of course, when the customer requests the retrieval of the object itself, the management section 4 does not search the stored information, but searches the stored objects to retrieve the requested object.

When searching for the deposited article based on the storage list of Figure 5, a different search method is employed.

The management section 4 searches the storage list of Figure 5 for the deposit ID 2193844. If the same deposit ID is found, then the data field is examined to see if it indicates "YES" or "NO".

If "YES", it means that the electronic data of the requested object is stored; therefore, the management section 4 directs the electronic warehouse 6 to retrieve the electronic data from the storage location E091-478A. The retrieved electronic data is transmitted to the hospital A.

In this search method, when the customer requests the retrieval of a stored object, the management section first checks whether or not the electronic data representing the requested object is stored; therefore, if both the requested object and its electronic data are stored, the electronic data is preferentially selected. Only when the electronic data does not exist, the requested object is retrieved from the object warehouse and converted by the conversion section 7 into electronic data.

Of course, when the customer requests the retrieval of the object itself, the management section 4 does not check the presence or absence of the electronic data of the object, but directs the object warehouse 5 to retrieve the requested object.

In either method described above, when the customer requests the retrieval of an object, if its electronic data is already stored, the electronic data is transmitted to the customer. This eliminates the need to repeat the same conversion job (the job of digitizing the X-ray photograph).

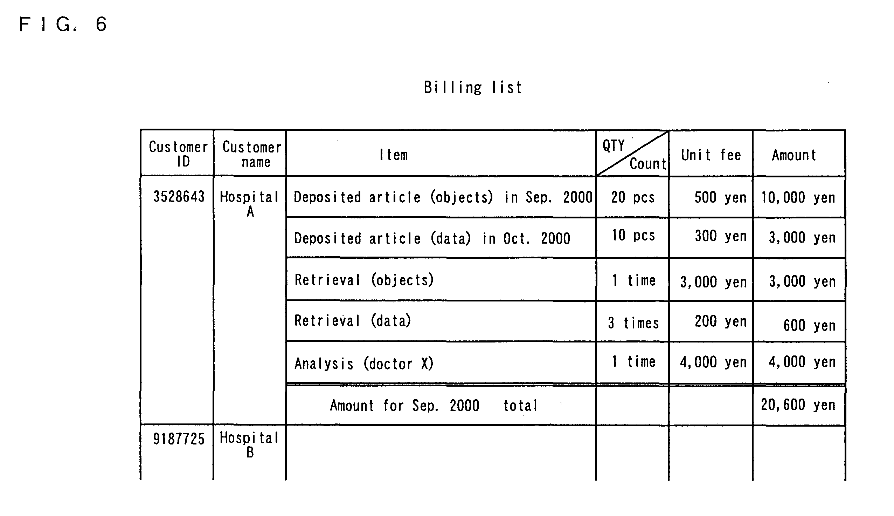

### <Explanation of the Billing Method (Figure 6)>

Figure 6 shows a billing list according to this embodiment.

The storage system 2 bills the customer for such services as storing objects and information entrusted for storage, sending stored objects (or their electronic versions) or information to the customer in accordance with retrieval requests received from the customer, and providing analysis service by a third party doctor at the request of the customer.

Figure 6 shows the contents of a bill for September 2000 that the storage system 2 sends to the hospital A.

The hospital A entrusted the storage of 20 objects and 10 pieces of information (data) to the storage system 2 in September 2000. The storage system 2 bills the customer according to the number of articles stored. Different per article fees can be charged for the storage of objets and the storage of information, as shown in Figure 6. The difference arises primarily from the difference in storage cost.

The calculation of the storage period starts from the time that the customer sends an object (or information) for entrustment to the storage system 2 or from the time that personnel from the storage system 2 visits the customer and receives the object, etc. for entrustment.

When there are a first storage place within a large hospital as a customer and a second storage place at a remote location, as shown in Figure 2, the calculation of the storage period may be started from the time that the customer deposits the object, etc. with the first storage place or from the time that the object, etc. is transferred from the first storage place to the second storage place.

The hospital A retrieved an object once and information three times in September 2000. The storage system 2 bills the customer for charges proportional to the number of object retrievals and information retrievals.

The per retrieval charge may be made the same for an object retrieval as for an information retrieval, or may be set differently as shown in Figure 6.

Retrieval (object) "1" means that the hospital A requested the retrieval of an X-ray photograph as an object once. In the present embodiment, when electronic data (bit mapped image data generated by the digitizer) of an X-ray photograph is transmitted to the hospital A, the charge is made based on the record "Retrieval (data)". Whether to charge the retrieval of the electronic data of an X-ray photograph as the retrieval of an object or the retrieval of information is optional.

The hospital A requested once in September the analysis of an X-ray photograph by a doctor X (see the record "Analysis (doctor X)", and the storage system charges the fee to the hospital A. A detailed procedure will be described later.

Analysis service means that the hospital A requests the analysis of an X-ray photograph, etc. by an experienced doctor outside the hospital.

The storage system 2 pays the analysis fee to the analyzing doctor on behalf of the client such as the hospital A, and charges the analysis fee to the client such as the hospital A on behalf of the analyzing doctor. Generally, the charged fee includes a commission for the intermediary service provided by the storage system 2.

In the prior art method, the hospital as the client paid the analysis fee directly to the analyzing doctor for each analysis.

In the present embodiment, since the storage system 2 provides the intermediary service, not only can the time and labor for sending an X-ray photograph, etc. be greatly saved, but, as will be described later, the time and labor involved in payment and billing can also be saved greatly since the payment and billing are made by grouping many items together rather than processing the payment and billing of the analysis fee on an item by item basis.

Besides the billing method explained with reference to Figure 6, another billing method may also be employed as will be explained in connection with an embodiment described later.

For example, clinic C, which does not entrust its objects or information to the storage system 2 for storage, may request the retrieval of a leg photograph of a patient S which a hospital B entrusted for storage. In such a case, the storage system 2 charges the expense only for the retrieval to the clinic C.

Likewise, patient P (who does not contract with the storage system 2 for the storage of his or her object or information) may request the retrieval of his or her leg photograph. In such a case, the storage system 2 charges the expense only for the retrieval to the patient P.

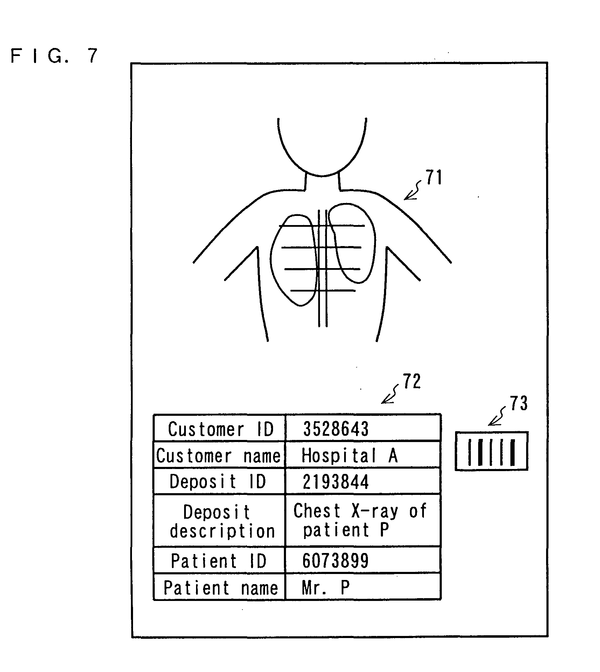

### <Explanation of Object Storage (Figure 7)>

Figure 7 shows the patient P's chest X-ray photograph stored in the object warehouse 5 of the present embodiment.

The chest X-ray photograph includes the chest X-ray photograph 71 and an identification label 72 and bar code 73 attached to the photograph.

The identification label 72 includes customer ID, customer name, deposit ID, deposited article name (description), patient ID, and patient name. Preferably, the customer hospital A enters the customer name, deposited article name, and patient name on the identification label, and attaches the identification label 72 to the X-ray photograph. The storage system 2 enters other items such as the customer ID. Since X-ray photographs look alike to a third party, the identification label 72 is attached to prevent the patient's X-ray photograph from being mistaken for another.

The bar code 73 contains the same information as that entered on the identification label 72. The bar code 73 alone may be attached to the chest X-ray photograph, and the identification label 72 may be omitted. The identification label 72 alone may be attached to the chest X-ray photograph, and the bar code 73 may be omitted.

Preferably, not only the bar code, which provides reliable electronic identification, but also the identification label should also be attached to enable visual identification by humans, ensuring the elimination of the possibility of mistaking the photograph for another patient's photograph.

Many X-ray imaging machines automatically superimpose characters such as the patient name and numerals such as ID on the X-ray photograph produced. Preferably, the label is produced by automatically reading the superimposed characters and numerals such as ID, and the label thus produced is attached to the X-ray photograph. By so doing, errors can be eliminated perfectly.

When a request to retrieve the chest X-ray photograph of the patient P is received from the hospital A, the chest X-ray photograph is converted into electronic data (bit mapped image data), and the bit mapped image data is transmitted to the hospital A.

In the present embodiment, when transmitting the chest X-ray photograph of the patient P to the hospital A by converting it into electronic data (bit mapped image data), the identification label 72 is always included in the bit mapped image data for transmission.

In the embodiment, the identification label 72 is always included in the bit mapped image data for transmission, but generally this is not always the case.

When transmitting bit mapped image data to the hospital A, since the name and customer identifier of the hospital A and the name and deposit ID of the deposited article are attached to the image data for transmission, the identification label 72 need not necessarily be included in the bit mapped image data.

When the hospital A transmits a letter of retrieval request, together with the customer ID 3528643 and the deposit ID 2193844, to the storage system 2, the storage system 2 transmits the bit mapped image data of the chest X-ray photograph of the patient P to the hospital A.

The storage system 2 compares the deposit ID 2193844 of the requested X-ray photograph with the deposit ID contained in the bar code attached to the chest X-ray photograph of the patient P and, after verifying that they match, generates bit mapped image data by means of the conversion section and transmits the bit mapped image data to the hospital A. Usually, this should eliminate the possibility of mistakenly transmitting another patient's photograph, but even so the risk cannot be eliminated completely. There is also the possibility that a doctor at the hospital A may enter an incorrect deposit ID (for example, the deposit ID of the chest X-ray photograph of a patient T hospitalized in the same hospital A).

In the present embodiment, however, since the identification label 72 is always included in the bit mapped image data, the doctor at the hospital A never fails to see the identification label on the image data when he or she analyzes the bit mapped image data. In this way, the doctor can visually verify whether the received bit mapped image data matches the requested data.

Wrongly identifying medical data must be prevented by all means, since it could endanger the life of the patient. Such accidents can be prevented by the above method.

In the case of the information stored in the electronic warehouse 6, since retrieval of all information is automatically done under the control of a computer, eliminating the possibility of wrongly identifying information, and since the information is not a material object, neither the identification label 72 nor the bar code 73 such as shown in Figure 7 is not needed.

However, if the information is recorded on a recording medium such as a DVD, since the job of retrieving the DVD and playing it back on the player is done by human hands, it is preferable to attach the identification label 72 and bar code 73 such as shown in Figure 7 to the surface of the container of the DVD.

Alternatively, the DVD player reads the medium's unique ID recorded on the DVD medium, to verify that the correct DVD medium has been retrieved.

### <<Embodiment 4>>

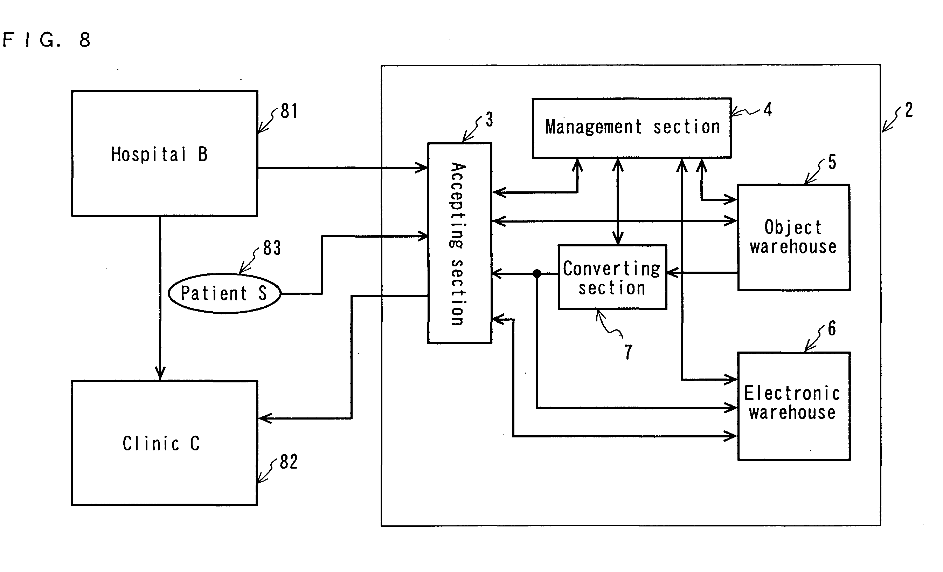

A fourth embodiment of the present invention will be described with reference to Figure 8.

In the fourth embodiment, the hospital B indicated at 81 and the clinic C indicated at 82 each entrust the storage system 2 with the storage of X-ray photographs, X-ray tomographic image data, electronic clinical charts, etc. they produce in their daily clinical activities. Now consider the case where a patient S 83 who has been in the hospital B 81 for the treatment of a burnt leg is discharged from the hospital and, after that, receives treatment at the clinic C 82 near the patient's home.

The storage system 2 comprises an accepting section 3, a management section 4, an object warehouse 5, an electronic warehouse 6, and a conversion section 7. The configuration of the storage system 2 is fundamentally the same as that of the first embodiment, and therefore, a description thereof will be omitted. The following description deals only with differences from the first embodiment.

When the patient S was in the hospital B 81, the hospital B 81 created an electronic clinical chart and took a photograph of the leg of the patient S 83 in the course of medical examination and treatment of the patient S 83 (Figures 4 and 5).

When the patient S 83 is discharged from the hospital B 81 and begins to receive treatment at the clinic C 82, it is desirable that the electronic clinical chart and the photograph of the patient S's leg, produced at the hospital B 81, be handed over to the clinic C 82 so that the clinic C 82 can give proper treatment to the patient S by understanding his or her treatment history. It is assumed that the hospital B 81 agreed to hand over the electronic clinical chart and the leg photograph of the patient S 83 to the clinic C 82.

In this case, according to the prior art, the hospital B 81 would terminate the entrustment of the storage of the electronic clinical chart and the photograph of the patient S's leg. The storage system 2 would return the leg photograph to the hospital B 81 by mail, and transmit the electronic clinical chart to the personal computer at the hospital B 81 via the Internet.

The hospital B 81 would deliver the returned leg photograph and the electronic clinical chart recorded on a floppy disk to the clinic C 82.

The clinic C 82 would send the received leg photograph and electronic clinical chart and the customer identifier of the clinic C, together with a letter of request for the storage of the object and data, to the storage system 2. The accepting section 3 in the storage system 2 would receive the object and data. The leg photograph would be stored again in the object warehouse 5, and the electronic clinical chart in the electronic warehouse 6.

The prior art has required a time and labor consuming procedure such as described above.

By contrast, in the fourth embodiment of the present invention, the electronic clinical chart and the leg photograph of the patient S can be transferred from the hospital B to the clinic C by following a much simpler procedure.

The hospital B transmits to the storage system 2 the customer ID 9187725 of the hospital B, the deposit IDs 5879825 and 1268354, and a letter of instruction instructing to transfer the electronic clinical chart and the leg photograph of the patient S to the clinic C.

The clinic C transmits to the storage system 2 the customer ID 0025864 of the clinic C, the deposit IDs 5879825 and 1268354 (reported in advance from the hospital B - in an alternative embodiment, the deposit IDs may be omitted), and a letter of consent to take over the storage of the electronic clinical chart and the leg photograph of the patient S.

The accepting section 3 in the storage system 2 receives these documents, verifies the respective customer IDs against the storage list (Figure 4 or 5), and changes the customer name and customer ID (customer identifier) for the stored electronic clinical chart and the leg photograph of the patient S from the hospital B to the clinic C (Figures 4 and 5).

The transfer is thus completed.

Preferably, the clinic C makes a customer registration with the storage system 2 prior to the above procedure. The name and customer identifier of the clinic C are registered on the storage list in the storage system 2.

When the storage list in the storage system 2 contains the patient ID (patient identifier) 4915134 (Figure 5), if the hospital B submits its own customer ID 9187725, the patient ID 4915134, and a letter of instruction instructing to transfer all the data and documents concerning the patient S to the clinic C, and the clinic C submits its own customer ID 0025864 and a letter of consent to take over the storage of all the data and documents concerning the patient S, then the management section 4 can convert the customer ID and customer name for all the objects and information bearing the patient ID of the patient S from the hospital B to the clinic C in a single operation. In this way, the transfer of the data and documents concerning the patient S can be accomplished without any omissions by following a simple procedure.

Furthermore, in the present embodiment, since the objects stored in the object warehouse 5 and the information stored in the electronic warehouse 6 are managed in a consolidating manner, the objects and information together can be transferred from the hospital B to the clinic C.

When the patient S transfers from the hospital B to the clinic C, the hospital B may refuse to deliver the electronic clinical chart and photograph of the patient S to the clinic C, as is often the case.

In such a case, according to the prior art, the clinic C has had no other way but to understand the treatment history by reading a letter from the hospital B that the patient S carried. Further, the clinic C has had to take a new photograph of the patient S' s leg and has had to create a new electronic clinical chart (or a paper clinical chart).

In this way, when the patient S transfers to another hospital, various difficulties have been involved in taking over the patient, incurring extra labor and expenses.

By contrast, according to the fourth embodiment, the taking over of the patient S from the hospital B to the clinic C can be accomplished in a much simpler procedure without the hospital B having to release the electronic clinical chart and the leg photograph of the patient S.

The hospital B transmits to the storage system 2 the customer ID 9187725 of the hospital B, the deposit IDs 5879825 and 1268354, and a letter of instruction permitting the clinic C to electronically view the electronic clinical chart and the leg photograph of the patient S.

The clinic C transmits to the storage system 2 the customer ID 0025864 of the clinic C, the deposit IDs 5879825 and 1268354 (in an alternative embodiment, the deposit IDs are not needed), and a letter of consent concerning the electronic viewing of the electronic clinical chart and the leg photograph of the patient S.

The accepting section 3 in the storage system 2 receives these documents, verifies the respective customer IDs against the storage list (Figure 4 or 5), and enters the name of the clinic C and the customer ID of the clinic C in the viewing permitted customer name field and the viewing permitted customer ID (customer identifier) field, respectively, for the electronic clinical chart of the patient S and the leg photograph of the patient S in the storage list. The viewing permitted customer name field and the viewing permitted customer ID field are new fields added to the right-hand edge of the storage list in Figure 4 or 5.

Preferably, the hospital B can select whether the clinic C should be permitted to retrieve only the electronically converted data of the leg photograph of the patient S (converted by the conversion section 7) or both the electronically converted data and the leg photograph itself.

The clinic C transmits a request requesting the retrieval of the electronic clinical chart and the leg photograph of the patient S, together with the customer ID 0025864 of the clinic C and the deposit IDs 5879825 and 1268354 (in an alternative embodiment, the deposit IDs are not needed), to the accepting section 3 in the storage system 2. The accepting section 3 transmits the electronic clinical chart and the electronic data of the leg photograph of the patient S to the clinic C after verifying that the received customer identifier matches the customer ID of the clinic C carried in the viewing permitted customer ID (customer identifier) field for the electronic clinical chart of the patient S and the leg photograph of the same patient in the storage list (Figure 4 or 5). Preferably, the accepting section 3 also verifies that the received customer name (clinic C) matches the viewing permitted customer name entered for the electronic clinical chart and the leg photograph of the patient S in the storage list.

With the above procedure, while the hospital B has control over the management of the electronic clinical chart and the leg photograph of the patient S, the clinic C can view the electronic clinical chart and the leg photograph of the patient S for examination.

In another embodiment, the hospital B transmits to the storage system 2 the customer ID 9187725 of the hospital B, the deposit IDs 5879825 and 1268354, and a letter of instruction agreeing to deliver a copy of the electronic clinical chart and a copy of the electronic data of the leg photograph of the patient S to the clinic C.

The clinic C transmits to the storage system 2 the customer ID 0025864 of the clinic C, the deposit IDs 5879825 and 1268354 (in an alternative embodiment, the deposit IDs are not needed), a letter of consent to receive the copy of the electronic clinical chart and the copy of the electronic data of the leg photograph of the patient S, and a letter of request to entrust the storage of these articles to the storage system 2.

After the accepting section 3 verifies the respective customer identifiers against the storage list (Figure 4 or 5), the leg photograph of the patient S entrusted by the hospital B for storage is retrieved from the object warehouse 5 under instruction from the management section 4, and its electronic data (bit mapped image data read by a digitizer) is generated by the conversion section 7. The leg photograph itself is returned to the object warehouse 5. The electronic data is newly stored in the electronic warehouse 6. The customer associated with the newly stored information, i.e., the electronic data, newly registered in the storage list, is the clinic C.

Likewise, a copy is made of the electronic clinical chart of the S patient stored in the electronic warehouse 6, and the copy is newly stored in the electronic warehouse 6. The copy is then registered in the storage list by being associated with the customer name, customer identifier, etc. of the clinic C.

The clinic C can now freely retrieve these pieces of information registered with its customer identifier attached thereto.

The storage system 2 charges the storage of these copies to the clinic C.

In another embodiment, a plurality of customer names and customer identifiers can be entered in the customer name field and the customer identifier field, respectively, in the storage list. The storage system 2 that received the above instruction letters, etc. newly registers the name and customer identifier of the clinic C in the record of the leg photograph, etc. in the storage list. The hospital B and the clinic C thus share the electronic clinical chart and the leg photograph of the patient S between them (however, the clinic C holds the ownership only of the electronic data). In this case, there is no need to produce copies of the electronic clinical chart and the electronic data of the leg photograph in order to create the information that the clinic C entrusts for storage.

Whether the clinic C has control over the management of only the electronic data of the leg photograph, not the leg photograph itself, can be easily identified by adding a field in the management list for identifying whether the clinic C has control over the management of the photograph itself or has control over the management only of its electronic data.

It should be noted that this becomes possible only when the management section 4 manages the object warehouse 5 and the electronic warehouse 6 in a consolidating manner. If the object warehouse 5 and the electronic warehouse 6 are managed separately from each other, an indication that the hospital does not have control over the management of the object itself but has control over the management of its electronic copy cannot be registered in a single management list.

In a further embodiment, the patient S 83 can transmit in person his or her patient ID 4915134 and a letter of instruction instructing to transfer his or her leg photograph and electronic clinical chart from the hospital B to the clinic C, to the accepting section 3 in the storage system 2. Preferably, the hospital B and the clinic C each transmit their customer ID, deposit IDs, and a letter of consent to the storage system 2.

The accepting section 3 verifies that the patient identifier attached to the letter of instruction matches the patient identifier (the patient ID in Figure 5) associated with the patient S's leg photograph, etc. in the storage list, and changes the customer name and customer identifier associated with the patient S's leg photograph, etc. to those of the clinic C.

In this way, according to the present embodiment, either the hospital B or the patient S can easily instruct to transfer the control over the management of the leg photograph, etc. to the clinic C.

### <<Embodiment 5>>

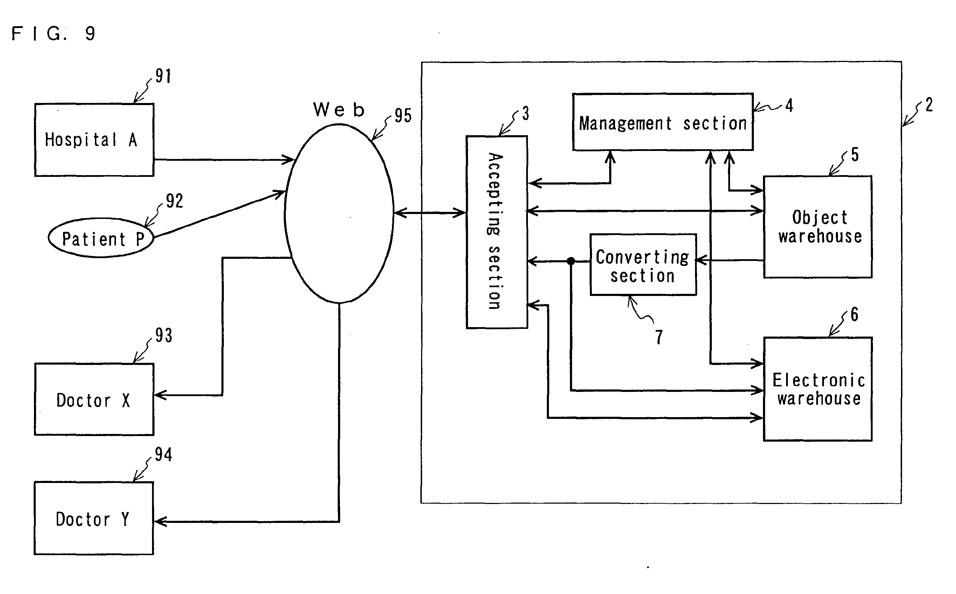

A fifth embodiment of the present invention will be described with reference to Figure 9.

While the prior art object or information storage system has been intended to simply store objects or information, the storage system 2 of the fifth embodiment provides not only storage service of objects and information but also intermediary service such as analysis service of medical information by a third party (for example, interpretation of X-ray tomographic image data).

Today, X-ray tomographic imaging machines are rapidly spreading in Japan and installed in many hospitals, but since analysis of X-ray tomographic image data (diagnosis based on image data) is an extremely difficult job, the market demand is growing for services that provide analysis and diagnosis of X-ray tomographic image data by experienced doctors when doctors at individual hospitals diagnose based on the X-ray tomographic image data.

According to the prior art, a hospital where a patient is hospitalized or is receiving treatment as an outpatient would find an experienced doctor through their personal connections and ask him for analysis and, after obtaining his consent, would send X-ray tomographic image data to the experienced doctor by mail. The experienced doctor would analyze and interpret the image data, write a medical certificate, and return the X-ray tomographic image data and the medical certificate to the hospital by mail. The hospital would then send an analysis fee to the experienced doctor.

In this way, entrusting image analysis and interpretation to an outside doctor would take a great deal of labor and time, and the reality is that an experienced doctor fit for the purpose cannot always be found, since such a doctor has to be found within a limited area.

The fifth embodiment illustrates a method by which a hospital where a patient is hospitalized or is receiving treatment as an outpatient can easily entrust the analysis of X-ray tomographic image data, etc. of that patient to a doctor outside the hospital.

In Figure 9, the patient P is hospitalized in the hospital A, and the hospital A entrusts the storage of the chest X-ray photograph of the patient P to the storage system 2 (Figure 4 or 5).

Suppose that the hospital A decided to ask an experienced doctor outside the hospital to analyze and diagnose the chest X-ray photograph of the patient P. The hospital A transmits its own customer ID 3528643, the deposit ID 2193844 of the chest X-ray photograph of the patient P, and a letter of request for the analysis of the chest X-ray photograph of the patient P, to the accepting section 3 in the storage system 2. The accepting section 3 verifies the customer ID of the hospital A against the storage list, and transmits to the hospital A a list of analysis service contracted doctors maintained in the management section 4.

The hospital A selects the doctor X from the list of analysis service contracted doctors.

Thereupon, the storage system 2 retrieves the chest X-ray photograph of the patient P from the object warehouse 5, converts it into electronic data (bit mapped image data) by means of the conversion section 7, and transmits the bit mapped image data to the doctor X 93.

At this time, the deposit ID is attached to the bit mapped image data for transmission, but any data that can identify the patient as an individual person, such as patient ID or patient name, is not attached. This is to prevent the leakage of the patient's secrecy. The secrecy can thus be ensured far more reliably than when the hospital would ask an outside doctor for analysis service through their personal connections.

The doctor X 93 analyzes and interprets the received bit mapped image data of the chest X-ray photograph, and writes a medical certificate. Preferably, the doctor X prepares the medical certificate, not on paper but in the form of an electronic file.

The doctor X transmits the medical certificate to the storage system 2.

The storage system 2 receives the medical certificate and transmits it to the hospital A. The hospital A can also entrust the storage of the medical certificate to the storage system 2.

The storage system 2 pays the analysis fee to the doctor X on a monthly basis, including fees for other analyses (for example, a fee for the analysis the doctor X did on behalf of the hospital B). The storage system 2 charges the analysis fee of the doctor X to the hospital A by including it in a monthly bill.

In this way, without physically transferring the chest X-ray photograph of the patient P, the hospital A can entrust the analysis of the image data to the desired experienced doctor through the intermediary service provided by the storage system 2.

The hospital A can also entrust the analysis of the image data to a plurality of doctors simultaneously. That is, the hospital A can select a plurality of doctors (doctor X and doctor Y) from the list of analysis service contracted doctors received from the storage system 2.

The storage system 2 transmits the electronic data of the chest X-ray photograph of the patient P to the doctor Y as well as to the doctor X. The doctor X and the doctor Y each analyze and interpret the received electronic data, write a medical certificate, and transmit the medical certificate to the storage system 2. The storage system 2 transmits the two medical certificates to the hospital A and, if the hospital A so desires, stores the two medical certificate.

In the prior art, it has not been possible to entrust the image analysis to more than one doctor since the X-ray photograph itself has had to be sent to the doctor. In the fifth embodiment, on the other hand, image analysis service by a plurality of doctors can be provided because electronic data is transmitted to the doctors for analysis.

It should be noted that the fifth embodiment can be easily implemented because the management section 4 manages the object warehouse and the electronic warehouse in a consolidating manner.

For example, suppose that the hospital A first requests the analysis of the image by the doctor X and, at a later date, additionally requests the analysis of the same X-ray photograph by the doctor Y. In this case, the storage system 2 need not retrieve the X-ray photograph from the object warehouse 5 and convert it again into electronic data by means of the conversion section 7, but need only retrieve the electronic data (produced when transmitting the data to the doctor X) from the electronic warehouse and transmit it to the doctor Y.

Furthermore, when a doctor at the hospital A searches the management list (only the records that match the customer name of the hospital A are extracted from the storage list of Figure 4 or 5 and displayed on the terminal screen at the hospital A), since X-ray photographs as objects and medical certificates as information (in electronic file form) are displayed simultaneously, the doctor can check all the data concerning the patient P at a glance and can easily understand the relationship between one piece of data and another.

In an alternative embodiment, the patient P transmits his or her patient ID 6073899, the deposit ID 2193844 of his or her chest X-ray photograph, and a letter of request requesting the analysis of the chest X-ray photograph by an outside doctor, to the storage system 2 (see Figure 5). The accepting section 3 verifies that the received patient ID matches the patient ID carried in the management list, and transmits to the patient P a list of analysis service contracted doctors maintained in the management section 4 in the same manner as earlier described.

The patient P selects the doctor X from the list of analysis service contracted doctors.

The storage system 2 transmits the electronic data (bit mapped image data) of the chest X-ray photograph of the patient P to the doctor X 93 in the same manner as earlier described.

The doctor X analyzes and interprets the received bit mapped image data of the chest X-ray photograph, and transmits the medical certificate to the storage system 2.

The storage system 2 receives the medical certificate and transmits it to the patient P.

The storage system 2 pays the analysis fee to the doctor X, and bills the patient P for the fee for the analysis done by the doctor X.

In this way, without physically transferring the chest X-ray photograph of the patient P, the patient P can entrust the analysis of the image data to the desired experienced doctor through the intermediary service provided by the storage system 2. In the prior art system, it has been next to impossible for a patient to find an experienced doctor outside the hospital by himself or herself and ask the doctor for the analysis, but according to the fifth embodiment, the patient can easily receive the analysis service of an experienced doctor.

As shown in Figure 9, the hospital A, the patient P, the doctor X, the doctor Y, and the storage system 2 are interconnected via the WEB 95 and can communicate information with each other via the Internet. Preferably, information communication between them is performed using cryptography such as ZIP.

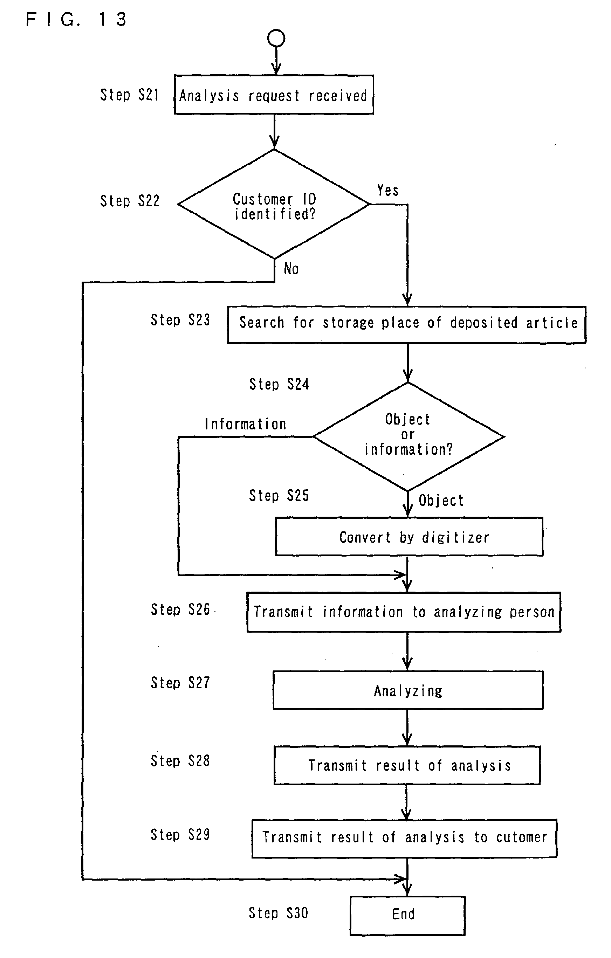

### <Explanation of Flow Chart for the Fifth Embodiment (Figure 13)>

Figure 13 is a flow chart for the fifth embodiment.

When a request for analysis of an object or information (for example, analysis of X-ray tomographic image data) is received from the customer, the request is processed in accordance with the procedure shown in Figure 13.

First, a request for analysis of an object or information is received from the customer (or patient) (step S21).

Next, the customer ID contained in the received data is identified. A search is made through the storage list (Figure 4 or 5), and if the same customer ID is found, the analysis request is granted; on the other hand, if the same customer ID is not found, the analysis request is rejected (step S22).

If the request is granted, a search is made for the storage place of the deposited article (object or information) that matches the deposit ID contained in the analysis request (step S23).

If the deposited article is information (for example, X-ray tomographic image data), the process proceeds to step S26, while if the deposited article is an object (for example, an X-ray photograph), the process proceeds to S25 (step S24).

If the deposited article is an object, the information contained in the deposited article is converted into first information by using a digitizer (step S25). Typically, an X-ray photograph is converted into bit mapped image data.

The information is transmitted to the analyzing person (step S26).

The analyzing person carries out the analysis (step S27). Typically, an experienced doctor analyzes and interprets the image data of the X-ray photograph or the X-ray tomographic image data.

The analyzing person transmits the result of the analysis (medical certificate) to the storage system (or directly to the customer) (step S28). When the result of the analysis is transmitted directly to the customer, the process terminates here.

When the analyzing person transmits the result of the analysis (medical certificate) to the storage system in Step S28, the storage system transmits the result of the analysis to the customer (step S29).

In the present invention, when the customer who entrusted the storage of an object (object or information) makes a request for retrieval of the stored object, the information contained in the object is converted into the first information, and the first information is sent to the customer via the Internet or the like, rather than sending the object itself to the customer.

Thus, the invention offers the advantage of achieving an object (object or information) management method that permits the customer to easily and quickly retrieve the information contained in the object entrusted for storage.

In the prior art management method, the cost of retrieval has been high because the object itself has had to be delivered from the storage warehouse to the customer and, after finishing the examination of the object, the customer has had to return the object to the warehouse.

The present invention offers the advantage of achieving a management method that can send the retrieved information from the warehouse to the customer at extremely low cost, particularly, by using a communication line such as the Internet, and that eliminates the need for the customer to return the information (no cost for return is incurred).

The present invention is applicable for storage of any kind of object, but is particularly suited as a storage method for storing data generated in the course of medical treatment and examination or preventive activities.

The invention offers the advantage of being able to achieve an object or information management method wherein a doctor, who is seeing a patient, can immediately retrieve image data of a previously taken X-ray photograph from an outside warehouse for display on the terminal screen at the hospital.

The present invention offers the advantage of being able to achieve a method for storing objects and information in a consolidating manner. As a result, the hospital can obtain a list of all the data (including objects and information) concerning the patient S, and can thus easily compare and study the data created in the past.

Furthermore, since the first information, once generated, is stored in the information warehouse (electronic warehouse), when the customer requests the retrieval of the same object for a second time, the first information stored in the electronic warehouse can be immediately transmitted to the customer without again converting the object into the first information. This saves the labor and time that would otherwise have to be expended for the conversion.

The invention thus offers the advantage of achieving an efficient object or information management method by linking the object warehouse and the electronic warehouse in coordinating fashion.

The invention also offers the advantage of achieving an object or information management method that enables high speed communications between the customer and the remotely located storage system (including a warehouse) using a communication line, thereby allowing the customer to quickly retrieve the stored information, etc.

The invention offers the advantage of achieving an object or information management method that can ensure secrecy of customer information by encrypting the data for transmission.

In the object or information method for storing, in particular, data that hospitals, etc. generate in the course of their medical treatment and examination or preventive activities, it is extremely important to verify the correctness of information because wrongly identifying information could endanger the life of the patient.

The present invention offers the advantage of achieving an object or information management method that can easily verify the correctness of the received information by visual means.

The invention also offers the advantage of being able to achieve an object or information method including the billing method.

In an object or information management method in which once the retrieval of an object is requested, the object is converted into first information and the first information is stored in the electronic warehouse, the present invention offers the advantage of being able to achieve an efficient object or information management method wherein when the first information is already stored, the first information, rather than the object itself, is retrieved from the warehouse (except when the retrieval of the object itself is requested), thereby eliminating the need to repeat the same conversion work (the generation of the first information).

In the object or information management method of the invention, the customer who entrusted the storage of an object to a storage company or the like can select whether to retrieve the object itself or the information contained in the object.

The invention thus offers the advantage of achieving an object or information management method that allows the customer to select the object or information for retrieval according to the needs of the customer.

When the customer requests the retrieval of the object itself, the conversion work (the generation of the first information) is not performed. This offers the advantage of achieving an efficient object or information management method by avoiding time-wasting work.

The ownership of the stored object belongs to the customer, and the storage company is bound to secrecy. In particular, it is important to protect the secrecy of objects, etc. entrusted by a hospital, etc. for storage.

The present invention offers the advantage of achieving an object or information management method that can ensure the secrecy of the customer.

In the case of objects or information generated in the course of medical treatment and examination or preventive activities, the probability of the generated data being retrieved is high shortly after they are generated, but the probability tends to decrease rapidly as the time elapses.

The present invention offers the advantage of achieving an object or information management method that provides enhanced convenience by changing the storage place of information (from the first storage place to the second storage place) depending on the probability of retrieval.

The present invention offers the advantage of achieving an object or information management method that provides service to entrust, at the request of the customer, the analysis of a stored object or information (for example, an X-ray photograph) to a third party.

The invention thus offers the advantage of being able to provide analysis service of, among others, data (objects or information) generated in the course of medical treatment and examination or preventive activities. In recent years, with advances in medical technologies, medical practitioners have become more and more specialized. In this context, if a general doctor can easily obtain a medical certificate from an expert doctor having a high analysis skill (for example, the interpreting of X-ray photographs or tomographic image data), it not only serves in the interest of the customer, but also greatly benefits the patient.

The present invention offers the advantage of achieving an object or information management method that can send the data for analysis to the doctor in a short time and that allows the customer to obtain the result of the analysis (medical certificate) in a short time.

The invention also offers the advantage of achieving an object or information management method that provides the service in which the analysis fee is paid to the doctor on behalf of the customer, together with the fees for analyses the doctor did for other customers.

The customer, on the other hand, pays the analysis fee to the storage company together with the monthly storage charge. This saves the customer the extra time and labor involved in the payment.

The present invention also offers the advantage of achieving an object or information management method that allows the customer to select a desired doctor from among a plurality of doctors and request the desired doctor for the analysis.

The present invention is particularly effective when the data to be analyzed is data measured on a patient, and when the patient (the second customer) wishes to entrust the analysis of his or her own measurement data (object or the second information) to an experienced doctor outside the hospital where the patient is hospitalized or is being treated as an outpatient.

The invention thus offers the advantage of achieving an object or information management method wherein, at the request of a patient, the analysis of an object concerning the patient (for example, an X-ray photograph of the patient), which the customer entrusted for storage, can be entrusted to a third party.

The patient can receive analysis service from an experienced doctor just by specifying the data to be analyzed (for example, an X-ray photograph) and submitting a request to the storage company to request the analysis by a third party (the patient may specify a particular doctor for the analysis).

Though the deposit ID is attached to the bit mapped image data for transmission, any data that can identify the patient as an individual person, such as patient ID or patient name, is not attached in order to protect the patient's secrecy.

The invention thus offers the advantage of achieving an object or information management method that can reliably protect the secrecy of the patient as an individual.

Since the storage company already has the requested data in the storage and also has a digitizer for X-ray photographs , etc., the storage company can send the data to the experienced doctor in a short time, and the patient can obtain the result of the analysis (medical certificate) in a short time.

The invention also offers the advantage of achieving an object or information management method that provides the service in which the analysis fee is paid to the doctor on behalf of the patient, together with the fees for analyses the doctor did for other customers.

The patient can accomplish the payment just by transferring the money for the analysis fee to the account of the storage company.

In the present invention, the storage company, on behalf of the customer, pays the analysis fee on a monthly basis to the doctor who provided the analysis service (the storage company contracts with the doctor for the analysis service). The storage company, in turn, charges the analysis fee to the customer together with the monthly storage fee (monthly billing).

The invention thus offers the advantage of achieving an object and information management method that provides analysis service the fees for which can be easily settled.

The present invention also offers the advantage of achieving an object or information management method wherein when a patient is transferred from hospital B to clinic C, the doctor at the clinic C can view on a terminal display at the clinic C the patient's previous X-ray photograph managed by the hospital B whenever the clinic C or the customer requests.

Although the invention has been described in some detail dealing with the preferred embodiments, the configuration details of any of the preferred embodiments disclosed herein may be changed or modified, and any changes in the combination or order of elements thereof can be accomplished without departing from the spirit and scope of the invention as set forth in the appended claims.

## Claims

1. An object or information management method comprising:
a storing step for storing an object of two-dimensional or three-dimensional shape received from a customer;
a first storing step for storing in a storage device a first identifier for identifying said customer and a second identifier for identifying said object by associating said first and second identifiers with said object;
an accepting step for accepting a request from said customer requesting retrieval of said object, said request containing therein said first identifier and said second identifier;
a step for comparing said first identifier contained in said request with said first identifier stored in said storage device, and for rejecting said request if said two first identifiers are not substantially the same;
a searching step for searching for said object based at least on said second identifier;
a converting step for converting information contained in said object into first information which is analog data or digital data; and
a sending step for sending said first information to said customer.

2. An object or information management method comprising:
a storing step for storing an object of two-dimensional or three-dimensional shape received from a customer, or for storing second information received from a customer in a storage device, said second information being analog data or digital data;
a first storing step for storing in a storage device a first identifier for identifying said customer and a second identifier for identifying said object or said second information;
an accepting step for accepting a request from said customer requesting retrieval of said object or said second information, said request containing therein said first identifier and said second identifier;
a step for comparing said first identifier contained in said request with said first identifier stored in said storage device, and for rejecting said request if said two first identifiers are not substantially the same;
a searching step for searching for said object or said second information based at least on said second identifier;
a converting step for converting information contained in said object into first information which is analog data or digital data, said converting step being performed when said customer is requesting the retrieval of said object; and
a sending step for sending said first information or said second information to said customer.

3. An object or information management method according to claim 1 or 2, further comprising the step of presenting said customer with an image display of a storage list showing both said stored object and said stored second information.

4. An object or information management method according to claim 1 or 2, wherein said accepting in said accepting step and said sending in said sending step are each performed by transmitting a digital data sequence over a communication line.

5. An object or information management method according to claim 4, wherein said digital data sequence is encrypted.

6. An information management method according to claim 1 or 2, wherein
said first information includes therein at least one of visual indications of customer name, customer identifier, attribute information of said object, identifier of said object, or an optical identifier having information consisting of at least one of said first and second identifiers, or
said second information includes therein at least one of visual indications of customer name, customer identifier, attribute information of said second information, identifier of said second information, or an optical identifier having information consisting of at least one of said first and second identifiers.

7. An information management method according to claim 1 or 2, further including:
a step for counting the number of times that said customer retrieves said object, said first information, or said second information; and
a step for billing said customer for charges including a charge corresponding to said number of times.

8. An information management method according to claim 1 or 2, wherein
when the information contained in said object is converted into said first information, said method further includes:
a second storing step for storing said first information in a storage device; and
a step for storing third information in a storage device by associating said third information with said object, said third information providing an indication of whether said first information into which the information contained in said object has been converted is stored or not, and wherein:
said searching step searches for said first information when said third information associated with said object to be searched for indicates that said first information is stored, and
when said first information is stored, said sending step reads out said stored first information and sends said readout first information.

9. An information management method according to claim 1 or 2, wherein
when the information contained in said object is converted into said first information, said method further includes a second storing step for storing said first information in a storage device, and wherein:
said searching step first searches for said first information and, if said first information is not found, then searches for said object, and
when said first information is stored, said sending step reads out said stored first information and sends said readout first information.

10. An information management method according to claim 1 or 2, wherein
when said object is already received from said customer, said accepting step allows said customer to select and specify whether said sending step should send said object or said first information,
when said customer specifies that said object be sent, said sending step sends said object to said customer,
when said customer specifies that said first information be sent, said sending step sends said first information to said customer, and
when said customer requests that said sending step send said object only, said converting step is not performed.

11. An information management method according to claim 1 or 2, further including:
a step for accepting a request from said customer for return of said object;
a step for returning said object to said customer;
a step for searching for said first information corresponding to said object; and
a step for erasing said first information if said first information is stored.

12. An information management method comprising:
a first storing step for storing second information received from a customer, said second information being analog data or digital data, a first identifier for identifying said customer, and a second identifier for identifying said second information, at an address having first location information in a first place;
a transmitting step for transmitting information including said first identifier, said second identifier, and said first location information, to a management section;
a first responding step for outputting said second information from a storage device located in said first place, if a request containing therein said first identifier and said second identifier and requesting retrieval of said second information is made by said customer;
a step for said management section to issue an instruction to transfer said second information from said first place to a second place when the period of storage in said first place has exceeded a predetermined period, or when the amount of information stored in said storage device in said first place has exceeded a predetermined amount, or when said customer requests said transfer;
a second storing step for storing said second information at an address having second location information in said second place;
a step for said management section to store therein said second location information by associating said second location information with said first identifier and said second identifier; and
a sending step for searching for said second information in accordance with an instruction from said management section, and sending said second information to said customer via a communication line, if a request containing therein said first identifier and said second identifier and requesting retrieval of said second information is received from said customer.

13. An information management method comprising:
a first depositing step for depositing an object received from a customer, or a medium received from said customer and having recorded thereon second information which is analog data or digital data, at an address having first location information in a first place;
a first storing step for transmitting information including a first identifier for identifying said customer, a second identifier for identifying said object or said medium, and said first location information, to a management section, and for storing said information in said management section;
an instructing step for said management section to issue an instruction to transfer said object or said medium from said first place to a second place when the period of deposit in said first place has exceeded a predetermined period, or when said object or said medium stored in said storage device in said first place has exceeded a predetermined quantity, or when said customer requests said transfer;
a second depositing step for transferring said object or said medium from said first place to said second place, and for depositing said object or said medium at an address having second location information in said second place;
a second storing step for said management section to store therein said second location information by associating said second location information with said first identifier and said second identifier; and
a sending step for searching for said object or said medium in accordance with an instruction from said management section, and sending first information, which is analog data or digital data obtained by converting information contained in said object, or said second information reproduced from said medium, to said customer via a communication line, if a request containing therein said first identifier and said second identifier and requesting retrieval of said object or said second information is received from said customer.

14. An information management method according to claim 12 or 13, wherein said management section includes a first computer comprising a communication device installed in said first place, and a second computer comprising a communication device installed in said second place.

15. An information management method according to claim 14, wherein said second computer has a first data space not accessible by said customer and a second data space accessible by said customer, and wherein said method further includes the step of allowing said customer to access data stored in said second data space via the Internet after said first identifier has been identified.

16. An object or information management method comprising:
a storing step for storing an object of two-dimensional or three-dimensional shape received from a customer;
a first storing step for storing in a storage device a first identifier for identifying said customer and a second identifier for identifying said object;
an accepting step for accepting a request from said customer requesting analysis of said object, said request containing therein said first identifier and said second identifier;
a step for comparing said first identifier contained in said request with said first identifier stored in said storage device, and for rejecting said request if said two first identifiers are not substantially the same;
a searching step for searching for said object based at least on said second identifier;
a converting step for converting information contained in said object into first information which is analog data or digital data;
a first sending step for sending said first information to an analyzing person;
a receiving step for receiving an analysis result transmitted from said analyzing person; and
a second sending step for sending said analysis result to said customer.

17. An object or information management method comprising:
a storing step for storing an object of two-dimensional or three-dimensional shape received from a customer, or for storing second information received from a customer in a storage device, said second information being analog data or digital data;
a first storing step for storing in a storage device a first identifier for identifying said customer and a second identifier for identifying said object or said second information;
an accepting step for accepting a request from said customer requesting analysis of said object or said second information, said request containing therein said first identifier and said second identifier;
a step for comparing said first identifier contained in said request with said first identifier stored in said storage device, and for rejecting said request if said two first identifiers are not substantially the same;
a searching step for searching for said object or said second information based at least on said second identifier;
a converting step for converting information contained in said object into first information which is analog data or digital data, said converting step being performed when said customer is requesting the analysis of said object;
a first sending step for sending said first information or said second information to an analyzing person;
a receiving step for receiving an analysis result transmitted from said analyzing person; and
a second sending step for sending said analysis result to said customer.

18. An object or information management method according to claim 16 or 17, further comprising:
a step for presenting a plurality of analyzing persons to said customer; and
a step for having said customer select at least one analyzing person from among said plurality of analyzing persons, and wherein:
said first information or said second information is sent to said selected analyzing person.

19. An object or information management method according to claim 16 or 17, wherein said method includes, instead of said receiving step and said second sending step, a third sending step for said analyzing person to send said analysis result directly to said customer.

20. An object or information management method according to claim 16 or 17, wherein said accepting in said accepting step, said sending in said first sending step, said second sending step, and said third sending step, and said receiving in said receiving step are each performed via a communication line.

21. An object or information management method according to claim 16 or 17, wherein
when the customer that sent said object or said second information is denoted as a first customer
said object, said first information, or said second information is an object or information concerning a second customer, and said analysis is carried out at the request of at least either one of said first and second customers.

22. An object or information management method according to claim 16 or 17, further comprising:
a step for paying an analysis fee to said analyzing person; and
a step for charging said analysis fee to said customer.

23. An object or information management method comprising:
a storing step for storing an object of two-dimensional or three-dimensional shape received from a first customer;
a first storing step for storing in a storage device a first customer identifier for identifying said first customer and a deposit identifier for identifying said object;
an accepting step for accepting an instruction containing therein said first customer identifier, said deposit identifier, and a second customer identifier for identifying a customer other than said first customer, said instruction instructing to grant a request for retrieval of said object if said request is received together with said second customer identifier and said deposit identifier;
a step for comparing said first customer identifier contained in said instruction with said first identifier stored in said storage device, and for rejecting said instruction if said two first customer identifiers are not substantially the same;
an accepting step for accepting said object retrieval request containing therein said second customer identifier and said deposit identifier; and
a step for permitting the retrieval of said object in accordance with said request if said second customer identifier contained in said request substantially matches said second customer identifier contained in said instruction.

24. An object or information management method comprising:
a storing step for storing an object of two-dimensional or three-dimensional shape received from a first customer, or for storing second information received from a first customer in a storage device, said second information being analog data or digital data;
a first storing step for storing in a storage device a first customer identifier for identifying said first customer and a deposit identifier for identifying said object or said second information;
an accepting step for accepting an instruction containing therein said first customer identifier, said deposit identifier, and a second customer identifier for identifying a customer other than said first customer, said instruction instructing to grant a request for retrieval of said object or said second information if said request is received together with said second customer identifier and said deposit identifier;
a step for comparing said first customer identifier contained in said instruction with said first identifier stored in said storage device, and for rejecting said instruction if said two first customer identifiers are not substantially the same;
an accepting step for accepting said object or second information retrieval request containing therein said second customer identifier and said deposit identifier; and
a step for permitting the retrieval of said object or said second information in accordance with said request if said second customer identifier contained in said request substantially matches said second customer identifier contained in said instruction.

25. An object or information management method comprising:
a storing step for storing an object of two-dimensional or three-dimensional shape received from a first customer, or for storing second information received from a first customer in a storage device, said second information being analog data or digital data;
a first storing step for storing in a storage device a first customer identifier for identifying said first customer and a deposit identifier for identifying said object or said second information;
an accepting step for accepting an instruction containing therein said first customer identifier, said deposit identifier, and a second customer identifier for identifying a customer other than said first customer, said instruction instructing to transfer a copy of said second information or a copy of first information generated from information contained in said object, to said customer having said second customer identifier;
a step for comparing said first customer identifier contained in said instruction with said first identifier stored in said storage device, and for rejecting said instruction if said two first customer identifiers are not substantially the same;
an accepting step for accepting a request containing therein said second customer identifier and said deposit identifier and requesting storage of the copy of said first information or said second information;
a step for permitting said customer having said second identifier at least to retrieve said first information or said second information for viewing if said second customer identifier contained in said request substantially matches said second customer identifier contained in said instruction; and
a step for charging said customer having said second identifier at least for the storage of said copy if said second customer identifier contained in said request substantially matches said second customer identifier contained in said instruction.

26. An object or information management method according to any one of claims 23 to 25, wherein
said object or said second information is associated with a customer having a third identifier, and
said first customer identifier is replaced by said third identifier.
